(19) Europäisches Patentamt
European Patent Office
Office européen des brevets

(11) EP 1 685 858 A2

(12) EUROPEAN PATENT APPLICATION

(43) Date of publication:
**02.08.2006 Bulletin 2006/31**

(51) Int Cl.:
***A61L 15/60*** *(2006.01)*

(21) Application number: **06250514.4**

(22) Date of filing: **31.01.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.02.2005 US 49074**

(71) Applicant: **KIMBERLY-CLARK WORLDWIDE, INC.
Neenah, WI 54956 (US)**

(72) Inventors:
- **Laumer, Jason Matthew
Appleton
Wisconsin 54915 (US)**
- **Herfert, Norbert
Charlotte
North Carolina 28266 (US)**
- **Miatudila, Ma-Ikay Kikama
Monroe
North Caroline
28110 (US)**
- **Mitchell, Michael A.
Waxhaw, North Caroline
28173 (US)**

(74) Representative: **Beacham, Annabel Rose
Frank B. Dehn & Co.
St Bride's House
10 Salisbury Square
London EC4Y 8JD (GB)**

# (54) Absorbent articles comprising polyamine-coated superabsorbent polymers

(57) An absorbent article comprises a fluid pervious topsheet, a fluid impervious backsheet which may be joined to the topsheet, and an absorbent core positioned between the topsheet and the backsheet. Additionally, the absorbent core comprises at least a superabsorbent material which contains at least a base polymer that has a pH less than six and has a surface coating comprising a polyamine. The result is an absorbent article which exhibits improved performance as well as greater comfort and confidence among the user.

EP 1 685 858 A2

## Description

### BACKGROUND

Absorbent articles are useful for absorbing many types of fluids, including fluids secreted or eliminated by the human body. Superabsorbent materials are frequently used in absorbent articles to help improve the absorbent properties of such articles. Superabsorbent materials are generally polymer based and are available in many forms, such as powders, granules, microparticles, films and fibers, for example. Upon contact with fluids, such superabsorbent materials swell by absorbing the fluids into their structures. In general, superabsorbent materials can quickly absorb fluids insulted into such articles, and can retain such fluids to prevent leakage and help provide a dry feel even after fluid insult.

There is continuing effort to improve the performance of such absorbent articles, especially at high levels of fluid saturation, to thereby reduce the occurrence of leakage. This is particularly significant when such articles are subjected to repeated fluid insults during use. This has become an increasing challenge as recent efforts in absorbent article design have generally focused on using higher concentrations of superabsorbent material and less fluff fibers to make the absorbent structures thinner and denser. However, notwithstanding the increase in total absorbent capacity obtained by increasing the concentration of superabsorbent material, such absorbent articles may still nevertheless leak during use. Such leakage may in part be the result of the absorbent core having an insufficient intake rate (i.e., the rate at which a fluid insult can be taken into and entrained within the absorbent core for subsequent absorption by the superabsorbent material) due to lack of available void volume. Therefore, there is a desire for an absorbent article which contains high levels of superabsorbent materials and which maintains a sufficient intake rate.

Additionally, increasing the performance of one absorbent property of an absorbent article can often result in an adverse effect on other absorbent properties. For example, as alluded to above, an increase in fluid intake rate can often result in a decrease in capacity. While an increase in fluid intake rate is generally desirable, a corresponding decrease in capacity is generally undesirable. Therefore, there is an additional desire for an absorbent article which exhibits an improved fluid intake rate without adversely affecting other absorbent properties such as capacity. Furthermore, there is a desire to accomplish such an improvement without resorting to complex, capital-intensive absorbent fabrication processes or additional non-absorbent binder fiber components. Such means should be compatible with conventional, low cost, efficient air-forming equipment that is widely used in the industry and integrated in the absorbent article manufacturing process.

### SUMMARY

The present invention concerns an absorbent article, suitably a disposable absorbent article, such as a training pant. More particularly, the absorbent article comprises a topsheet, a backsheet and an absorbent core positioned between the topsheet and the backsheet. Additionally, the absorbent core comprises at least a superabsorbent material which contains at least a base polymer that has a pH of less than six, and has a surface coating comprising a polyamine. The result is an absorbent article which exhibits improved performance as well as greater comfort and confidence among the user.

Numerous other features and advantages of the present invention will appear from the following description. In the description, reference is made to exemplary embodiments of the invention. Such embodiments do not represent the full scope of the invention. Reference should therefore be made to the claims herein for interpreting the full scope of the invention.

### FIGURES

The foregoing and other features, aspects and advantages of the present invention will become better understood with regard to the following description, appended claims and accompanying drawings where:

FIG 1 is a perspective view of one embodiment of an absorbent article that may be made in accordance with the present invention;

FIG 2 is a plan view of the absorbent article shown in FIG 1 with the article in an unfastened, unfolded and laid flat condition showing the surface of the article that faces the wearer when worn and with portions cut away to show underlying features;

FIG 3 is a schematic diagram of one version of a method and apparatus for producing an absorbent core;

FIG 4 is a cross-sectional side view of a layered absorbent core according to the present invention;

FIG 5 is a partially cut away top view of a saturated capacity tester;

FIG 6 is a side view of a Saturated Capacity tester;

FIG 7 is a rear view of a Saturated Capacity tester;

FIG 8 is a top view of the test apparatus employed for the Fluid Intake Flux Test; and

FIG 9 is a side view of the test apparatus employed for the Fluid Intake Flux Test.

Repeated use of reference characters in the present specification and drawings is intended to represent the same or analogous features or elements of the present invention.

**DEFINITIONS**

It should be noted that, when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

The term "absorbent article" generally refers to devices which can absorb and contain fluids. For example, personal care absorbent articles refer to devices which are placed against or near the skin to absorb and contain the various fluids discharged from the body. The term "disposable" is used herein to describe absorbent articles that are not intended to be laundered or otherwise restored or reused as an absorbent article after a single use. Examples of such disposable absorbent articles include, but are not limited to, personal care absorbent articles, health/medical absorbent articles, and household/industrial absorbent articles.

The term "coform" is intended to describe a blend of meltblown fibers and cellulose (e.g. wood) fibers that is formed by air forming a meltblown polymer material while simultaneously blowing air-suspended cellulose fibers into the stream of meltblown fibers. The coform material may also include other materials, such as superabsorbent materials. The meltblown fibers containing cellulose fibers are collected on a forming surface, such as provided by a foraminous belt. The forming surface may include a gas-pervious material, such as spunbonded fabric material, that has been placed onto the forming surface.

The terms "elastic," "elastomeric" and "elastically extensible" are used interchangeably to refer to a material or composite that generally exhibits properties which approximate the properties of natural rubber. The elastomeric material is generally capable of being extended or otherwise deformed, and then recovering a significant portion of its shape after the extension or deforming force is removed.

The term "extensible" refers to a material that is generally capable of being extended or otherwise deformed, but which does not recover a significant portion of its shape after the extension or deforming force is removed.

The term "fluid impermeable", when used to describe a layer or laminate, means that fluid such as water or bodily fluids will not pass substantially through the layer or laminate under ordinary use conditions in a direction generally perpendicular to the plane of the layer or laminate at the point of fluid contact.

The term "health/medical absorbent article" includes a variety of professional and consumer health-care products including, but not limited to, products for applying hot or cold therapy, medical gowns (i.e., protective and/or surgical gowns), surgical drapes, caps, gloves, face masks, bandages, wound dressings, wipes, covers, containers, filters, disposable garments and bed pads, medical absorbent garments, underpads, and the like.

The term "household/industrial absorbent articles" include construction and packaging supplies, products for cleaning and disinfecting, wipes, covers, filters, towels, disposable cutting sheets, bath tissue, facial tissue, nonwoven roll goods, home-comfort products including pillows, pads, mats, cushions, masks and body care products such as products used to cleanse or treat the skin, laboratory coats, cover-alls, trash bags, stain removers, topical compositions, pet care absorbent liners, laundry soil/ink absorbers, detergent agglomerators, lipophilic fluid separators, and the like.

The terms "hydrophilic" and "wettable" are used interchangeably to refer to a material having a contact angle of water in air of less than 90 degrees. The term "hydrophobic" refers to a material having a contact angle of water in air of at least 90 degrees. For the purposes of this application, contact angle measurements are determined as set forth in Robert J. Good and Robert J. Stromberg, Ed., in "Surface and Colloid Science - Experimental Methods," Vol. II, (Plenum Press, 1979).

The term "insult target zone" refers to an area of an absorbent core where it is particularly desirable for the majority of a fluid insult, such as urine, menses, or bowel movement, to initially contact. In particular, for an absorbent core with one or more fluid insult points in use, the insult target zone refers to the area of the absorbent core extending a distance equal to 15% of the total length of the core from each insult point in both directions.

The term "materials" when used in the phrase "superabsorbent materials" refers generally to discrete units. The units can comprise particles, granules, fibers, flakes, agglomerates, rods, spheres, needles, particles coated with fibers or other additives, pulverized materials, powders, films, and the like, as well as combinations thereof. The materials can have any desired shape such as, for example, cubic, rod-like, polyhedral, spherical or semi-spherical, rounded or semi-rounded, angular, irregular, etc. Additionally, superabsorbent materials may be composed of more than one type of material.

The term "meltblown fibers" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers.

The terms "nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblowing processes, spunbonding processes, air laying processes, and bonded-carded-web processes. The basis weight of nonwoven fabrics is usually expressed in ounces of material per square yard (osy) or grams per square meter (gsm) and the fiber diameters are usually expressed in microns. (Note that to convert from osy to gsm, multiply osy by 33.91.)

The term "personal care absorbent article" includes, but is not limited to, absorbent articles such as diapers, diaper pants, baby wipes, training pants, absorbent underpants, child care pants, swimwear, and other disposable garments; feminine care products including sanitary napkins, wipes, menstrual pads, menstrual pants, panty liners, panty shields, interlabials, tampons, and tampon applicators; adult-care products including wipes, pads such as breast pads, containers, incontinence products, and urinary shields; clothing components; bibs; athletic and recreation products; and the like.

The term "polymers" includes, but is not limited to, homopolymers, copolymers, such as for example, block, graft, random and alternating copolymers, terpolymers, etc. and blends and modifications thereof. Furthermore, unless otherwise specifically limited, the term "polymer" shall include all possible configurational isomers of the material. These configurations include, but are not limited to isotactic, syndiotactic and atactic symmetries.

The terms "spunbond" and "spunbonded fiber" refer to fibers which are formed by extruding filaments of molten thermoplastic material from a plurality of fine, usually circular, capillaries of a spinneret, and then rapidly reducing the diameter of the extruded filaments.

The term "stretchable" refers to materials which may be extensible or which may be elastically extensible.

The terms "superabsorbent" and "superabsorbent materials" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In contrast, "absorbent materials" are capable, under the most favorable conditions, of absorbing at least 5 times their weight of an aqueous solution containing 0.9 weight percent sodium chloride.

These terms may be defined with additional language in the remaining portions of the specification.

## DETAILED DESCRIPTION

The present invention concerns an absorbent article, suitably a disposable absorbent article, such as a training pant. More particularly, the absorbent article comprises a topsheet which may be fluid pervious, a backsheet which may be fluid impervious and that may be joined to the topsheet, and an absorbent core positioned between the topsheet and the backsheet. Additionally, the absorbent core comprises at least a superabsorbent material which contains at least a base polymer that has a pH of less than six and has a surface coating comprising a polyamine. The result is an absorbent article which exhibits improved performance as well as greater comfort and confidence among the user.

In some embodiments, at least the topsheet can be stretchable, while in other embodiments, at least the backsheet can be stretchable. In still other embodiments, the absorbent core can be stretchable. The absorbent article may also include other components, such as fluid wicking layers, intake layers, surge layers, distribution layers, transfer layers, barrier layers, wrapping layers and the like, as well as combinations thereof.

Referring to FIGs 1 and 2 for exemplary purposes, a training pant which may incorporate the present invention is shown. It is understood that the present invention is suitable for use with various other absorbent articles, including but not limited to other personal care absorbent articles, health/medical absorbent articles, household/industrial absorbent articles and the like without departing from the scope of the present invention.

Various materials and methods for constructing training pants are disclosed in PCT Patent Application WO 00/37009 published June 29, 2000 by A. Fletcher et al.; U.S. Patents 4,940,464 to Van Gompel et al.; 5,766,389 to Brandon et al. and 6,645,190 to Olson et al.

FIG 1 illustrates a training pant in a partially fastened condition, and FIG 2 illustrates a training pant in an opened and unfolded state. The training pant defines a longitudinal direction 48 that extends from the front of the training pant when worn to the back of the training pant. Perpendicular to the longitudinal direction 48 is a lateral direction 49.

The pair of training pants defines a front region 22, a back region 24 and a crotch region 26 extending longitudinally between and interconnecting the front and back regions. The pant also defines an inner surface adapted in use (e.g., positioned relative to the other components of the pant) to be disposed toward the wearer, and an outer surface opposite the inner surface. The training pant has a pair of laterally opposite side edges and a pair of longitudinally opposite waist edges.

The illustrated pant 20 may include a chassis 32, a pair of laterally opposite front side panels 34 extending laterally outward at the front region 22 and a pair of laterally opposite back side panels 134 extending laterally outward at the back region 24.

Referring to FIGs 1 and 2, the chassis 32 includes a backsheet 40 and a topsheet 42 that may be joined to the backsheet 40 in a superimposed relation therewith by adhesives, ultrasonic bonds, thermal bonds or other conventional techniques. The chassis 32 may further include an absorbent core 44 such as shown in FIG 2 disposed between the backsheet 40 and the topsheet 42 for absorbing fluid body exudates exuded by the wearer, and may further include a pair of containment flaps 46 secured to the topsheet 42 or the absorbent core 44 for inhibiting the lateral flow of body exudates.

The backsheet 40, the topsheet 42 and the absorbent core 44 may be made from many different materials known to those skilled in the art. All three layers, for instance, may be extensible and/or elastically extensible. Further, the stretch properties of each layer may vary in order to control the overall stretch properties of the product.

The backsheet 40, for instance, may be breathable and/or may be fluid impermeable. The backsheet 40 may be constructed of a single layer, multiple layers, laminates, spunbond fabrics, films, meltblown fabrics, elastic netting, microporous webs or bonded-carded-webs. The backsheet 40, for instance, can be a single layer of a fluid impermeable material, or alternatively can be a multilayered laminate structure in which at least one of the layers is fluid impermeable.

The backsheet 40 can be biaxially extensible and optionally biaxially elastic. Elastic non-woven laminate webs that can be used as the backsheet 40 include a non-woven material joined to one or more gatherable non-woven webs or films. Stretch Bonded Laminates (SBL) and Neck Bonded Laminates (NBL) are examples of elastomeric composites.

Examples of suitable nonwoven materials are spunbond-meltblown fabrics, spunbond-meltblown-spunbond fabrics, spunbond fabrics, or laminates of such fabrics with films, or other nonwoven webs. Elastomeric materials may include cast or blown films, meltblown fabrics or spunbond fabrics composed of polyethylene, polypropylene, or polyolefin elastomers, as well as combinations thereof. The elastomeric materials may include PEBAX elastomer (available from AtoFina Chemicals, Inc., a business having offices located in Philadelphia, Pennsylvania U.S.A), HYTREL elastomeric polyester (available from Invista, a business having offices located in Wichita, Kansas U.S.A.), KRATON elastomer (available from Kraton Polymers, a business having offices located in Houston, Texas, U.S.A.), or strands of LYCRA elastomer (available from Invista), or the like, as well as combinations thereof. The backsheet 40 may include materials that have elastomeric properties through a mechanical process, printing process, heating process or chemical treatment. For example, such materials may be apertured, creped, neck-stretched, heat activated, embossed, and micro-strained, and may be in the form of films, webs, and laminates.

One example of a suitable material for a biaxially stretchable backsheet 40 is a breathable elastic film/nonwoven laminate, such as described in U.S. Patent 5,883,028, to Morman et al. Examples of materials having two-way stretchability and retractability are disclosed in U.S. Patents 5,116,662 to Morman and 5,114,781 to Morman. These two patents describe composite elastic materials capable of stretching in at least two directions. The materials have at least one elastic sheet and at least one necked material, or reversibly necked material, joined to the elastic sheet at least at three locations arranged in a nonlinear configuration, so that the necked, or reversibly necked, web is gathered between at least two of those locations.

The topsheet 42 is suitably compliant, soft-feeling and non-irritating to the wearer's skin. The topsheet 42 is also sufficiently liquid permeable to permit liquid body exudates to readily penetrate through its thickness to the absorbent core 44. A suitable topsheet 42 may be manufactured from a wide selection of web materials, such as porous foams, reticulated foams, apertured plastic films, woven and non-woven webs, or a combination of any such materials. For example, the topsheet 42 may include a meltblown web, a spunbonded web, or a bonded-carded-web composed of natural fibers, synthetic fibers or combinations thereof. The topsheet 42 may be composed of a substantially hydrophobic material, and the hydrophobic material may optionally be treated with a surfactant or otherwise processed to impart a desired level of wettability and hydrophilicity.

The topsheet 42 may also be extensible and/or elastomerically extensible. Suitable elastomeric materials for construction of the topsheet 42 can include elastic strands, LYCRA elastics, cast or blown elastic films, nonwoven elastic webs, meltblown or spunbond elastomeric fibrous webs, as well as combinations thereof. Examples of suitable elastomeric materials include KRATON elastomers, HYTREL elastomers, ESTANE elastomeric polyurethanes (available from Noveon, a business having offices located in Cleveland, Ohio U.S.A.), or PEBAX elastomers. The topsheet 42 can also

be made from extensible materials such as those described in U.S. Patent 6,552,245 to Roessler et al. The topsheet 42 can also be made from biaxially stretchable materials as described in U.S. Patent 6,641,134 filed to Vukos et al.

The article 20 can optionally further include a surge management layer which may be located adjacent the absorbent core 44 and attached to various components in the article 20 such as the absorbent core 44 or the topsheet 42 by methods known in the art, such as by using an adhesive. In general, a surge management layer helps to quickly acquire and diffuse surges or gushes of liquid that may be rapidly introduced into the absorbent structure of the article. The surge management layer can temporarily store the liquid prior to releasing it into the storage or retention portions of the absorbent core 44. Examples of suitable surge management layers are described in U.S. Patents 5,486,166 to Bishop et al.; 5,490,846 to Ellis et al.; and 5,820,973 to Dodge et al.

The article 20 can further comprise an absorbent core 44. The absorbent core 44 may have any of a number of shapes. For example, it may have a 2-dimensional or 3-dimensional configuration, and may be rectangular shaped, triangular shaped, oval shaped, race-track shaped, I-shaped, generally hourglass shaped, T-shaped and the like. It is often suitable for the absorbent core 44 to be narrower in the crotch portion 26 than in the rear 24 or front 22 portion(s). The absorbent core 44 can be attached in an absorbent article, such as to the backsheet 40 and/or the topsheet 42 for example, by bonding means known in the art, such as ultrasonic, pressure, adhesive, aperturing, heat, sewing thread or strand, autogenous or self-adhering, hook-and-loop, or any combination thereof.

In some aspects, the absorbent core 44 can have a significant amount of stretchability. For example, the absorbent core 44 can comprise a matrix of fibers which includes an operative amount of elastomeric polymer fibers. Other methods known in the art can include attaching superabsorbent materials to a stretchable film, utilizing a nonwoven substrate having cuts or slits in its structure, and the like.

The absorbent core 44 can also include absorbent material, such as superabsorbent material and/or fluff. Additionally, the superabsorbent material can be operatively contained within a matrix of fibers, such as polymeric fibers. Accordingly, the absorbent core 44 can comprise a quantity of superabsorbent material and/or fluff contained within a matrix of fibers. In some aspects, the amount of superabsorbent material in the absorbent core 44 can be at least about 10% by weight of the core, such as at least about 30%, or at least about 60% by weight or at least about 80%, or between about 10% and about 80% by weight of the core to provide improved benefits. Optionally, the amount of superabsorbent material can be at least about 95-percent by weight of the core. In other aspects, the absorbent core 44 can comprise about 35-percent or less by weight fluff, such as about 25-percent or less, or 15-percent or less by weight fluff.

It should be understood that the present invention is not restricted to use with superabsorbent materials and/or fluff. In some aspects, the absorbent core 44 may additionally or alternatively include materials such as surfactants, ion exchange resin particles, moisturizers, emollients, perfumes, natural fibers, synthetic fibers, fluid modifiers, odor control additives, and combinations thereof. Alternatively, the absorbent core 44 can be or can include a foam.

In order to function well, the absorbent core 44 can have certain desired properties to provide improved performance as well as greater comfort and confidence among the user. For instance, the absorbent core 44 can have corresponding configurations of absorbent capacities, densities, basis weights and/or sizes which are selectively constructed and arranged to provide desired combinations of absorbency properties such as liquid intake rate, absorbent capacity, liquid distribution or fit properties such as shape maintenance and aesthetics. Likewise, the components can have desired wet to dry strength ratios, mean flow pore sizes, permeabilities and elongation values.

As mentioned above, the absorbent core 44 can optionally include elastomeric polymer fibers. The elastomeric material of the polymer fibers may include an olefin elastomer or a non-olefin elastomer, as desired. For example, the elastomeric fibers can include olefinic copolymers, polyethylene elastomers, polypropylene elastomers, polyester elastomers, polyisoprene, cross-linked polybutadiene, diblock, triblock, tetrablock, or other multi-block thermoplastic elastomeric and/or flexible copolymers such as block copolymers including hydrogenated butadiene-isoprene-butadiene block copolymers; stereoblock polypropylenes; graft copolymers, including ethylene-propylene-diene terpolymer or ethylene-propylene-diene monomer (EPDM) rubber, ethylene-propylene random copolymers (EPM), ethylene propylene rubbers (EPR), ethylene vinyl acetate (EVA), and ethylene-methyl acrylate (EMA); and styrenic block copolymers including diblock and triblock copolymers such as styrene-isoprene-styrene (SIS), styrene-butadiene-styrene (SBS), styrene-isoprene-butadiene-styrene (SIBS), styrene-ethylene/butylene-styrene (SEBS), or styrene-ethylene/propylene-styrene (SEPS), which may be obtained from Kraton Inc. under the trade designation KRATON elastomeric resin or from Dexco, a division of ExxonMobil Chemical Company (a business having offices located in Houston, Texas U.S.A.) under the trade designation VECTOR (SIS and SBS polymers); blends of thermoplastic elastomers with dynamic vulcanized elastomer-thermoplastic blends; thermoplastic polyether ester elastomers; ionomeric thermoplastic elastomers; thermoplastic elastic polyurethanes, including those available from Invista Corporation under the trade name LYCRA polyurethane, and ESTANE available from Noveon, Inc. (a business having offices located in Cleveland, Ohio U.S.A.); thermoplastic elastic polyamides, including polyether block amides available from AtoFina Chemicals, Inc. (a business having offices located in Philadelphia, Pennsylvania U.S.A.) under the trade name PEBAX; polyether block amide; thermoplastic elastic polyesters, including those available from E. I. Du Pont de Nemours Co., under the trade name HYTREL, and ARNITEL from DSM Engineering Plastics (a business having offices located in Evansville, Indiana, U.S.A.)

and single-site or metallocene-catalyzed polyolefins having a density of less than about 0.89 grams/cubic centimeter, available from Dow Chemical Co. (a business having offices located in Freeport, Texas U.S.A.) under the trade name AFFINITY; and combinations thereof.

As used herein, a tri-block copolymer has an ABA structure where the A represents several repeat units of type A, and B represents several repeat units of type B. As mentioned above, several examples of styrenic block copolymers are SBS, SIS, SIBS, SEBS and SEPS. In these copolymers the A blocks are polystyrene and the B blocks are a rubbery component. Generally, these triblock copolymers have molecular weights that can vary from the low thousands to hundreds of thousands, and the styrene content can range from 5% to 75% based on the weight of the triblock copolymer. A diblock copolymer is similar to the triblock, but is of an AB structure. Suitable diblocks include styrene-isoprene diblocks, which have a molecular weight of approximately one-half of the triblock molecular weight having the same ratio of A blocks to B blocks.

In desired arrangements, the polymer fibers can include at least one material selected from the group consisting of styrenic block copolymers, elastic polyolefin polymers and co-polymers and EVA/EMA type polymers.

In other particular arrangements, for example, the elastomeric material of the polymer fibers can include various commercial grades of low crystallinity, lower molecular weight metallocene polyolefins, available from ExxonMobil Chemical Company (a company having offices located in Houston, Texas, U.S.A.) under the VISTAMAXX trade designation. The VISTAMAXX material is believed to be metallocene propylene ethylene co-polymer. For example, in one aspect the elastomeric polymer can be VISTAMAXX PLTD 2210. In other aspects, the elastomeric polymer can be VISTAMAXX PLTD 1778. Another optional elastomeric polymer is KRATON blend G 2755 from Kraton Inc. The KRATON material is believed to be a blend of styrene ethylene-butylene styrene polymer, ethylene waxes and tackifying resins.

In some aspects, the elastomeric polymer fibers can be produced from a polymer material having a selected melt flow rate (MFR). In a particular aspect, the MFR can be up to a maximum of about 300. Alternatively, the MFR can be up to about 230 or 250. In another aspect, the MFR can be a minimum of not less than about 20. The MFR can alternatively be not less than about 50 to provide desired performance. The described melt flow rate has the units of grams flow per 10 minutes (g/10 min). The parameter of melt flow rate is well known, and can be determined by conventional techniques, such as by employing test ASTM D 1238 70 "extrusion plastometer" Standard Condition "L" at 230°C and 2.16 kg applied force.

As mentioned above, the polymer fibers of the absorbent core 44 can include an amount of a surfactant. The surfactant can be combined with the polymer fibers of the absorbent core in any operative manner. Various techniques for combining the surfactant are conventional and well known to persons skilled in the art. For example, the surfactant may be compounded with the polymer employed to form a meltblown fiber structure. In a particular feature, the surfactant may be configured to operatively migrate or segregate to the outer surface of the fibers upon the cooling of the fibers. Alternatively, the surfactant may be applied to or otherwise combined with the polymer fibers after the fibers have been formed.

The polymer fibers can include an operative amount of surfactant, based on the total weight of the fibers and surfactant. In some aspects, the polymer fibers can include at least a minimum of about 0.1 % by weight surfactant, as determined by water extraction. The amount of surfactant can alternatively be at least about 0.15% by weight, and can optionally be at least about 0.2% by weight to provide desired benefits. In other aspects, the amount of surfactant can be generally not more than a maximum of about 2% by weight, such as not more than about 1 % by weight, or not more than about 0.5% by weight to provide improved performance.

If the amount of surfactant is outside the desired ranges, various disadvantages can occur. For example, an excessively low amount of surfactant may not allow fibers, such as hydrophobic meltblown fibers, to wet with the absorbed fluid. In contrast, an excessively high amount of surfactant may allow the surfactant to wash off from the fibers and undesirably interfere with the ability of the absorbent core to transport fluid, or may adversely affect the attachment strength of the absorbent core 44 to the absorbent article 20. Where the surfactant is compounded or otherwise internally added to the polymer fibers, an excessively high level of surfactant can create conditions that cause a poor formation of the polymer fibers.

In some configurations, the surfactant can include at least one material selected from the group that includes polyethylene glycol ester condensates and alkyl glycoside surfactants. For example, the surfactant can be a GLUCOPON surfactant, available from Cognis Corporation (a business having offices located in Cincinnati, Ohio, U.S.A.) which can be composed of 40-percent water, and 60-percent D-glucose, decyl, octyl ethers and oligomerics.

In other aspects of the invention, the surfactant can be in the form of a sprayed-on surfactant comprising a water/surfactant solution which includes 16 liters of hot water (about 45 °C to 50 °C) mixed with 0.20 kg of GLUCOPON 220 UP surfactant available from Cognis Corporation and 0.36 kg of AHCHOVEL Base N-62 surfactant available from Uniqema (a business having offices located in New Castle, Delaware, U.S.A.). When employing a sprayed-on surfactant, a relatively lower amount of sprayed-on surfactant may be desirable to provide the desired containment of the superabsorbent material. Excessive amounts of the fluid surfactant may hinder the desired attachment of the superabsorbent material to the molten, elastomeric meltblown fibers, for example.

An example of an internal surfactant or wetting agent that can be compounded with the elastomeric fiber polymer can include a MAPEG DO 400 PEG (polyethylene glycol) ester, available from BASF (a business having offices located in Freeport, Texas, U.S.A.). Other internal surfactants can include a polyether, a fatty acid ester, a soap or the like, as well as combinations thereof.

The absorbent core 44 also includes a desired amount of superabsorbent material of the present invention. Superabsorbent materials typically are polymers of unsaturated carboxylic acids or derivatives thereof. These polymers are rendered water insoluble, but water swellable, by crosslinking the polymer with a di- or poly-functional internal crosslinking agent. These internally crosslinked polymers are at least partially neutralized and contain pendant anionic carboxyl groups on the polymer backbone that enable the polymer to absorb aqueous fluids, such as body fluids.

Superabsorbent materials are manufactured by known polymerization techniques, preferably by polymerization in aqueous solution by gel polymerization. The products of this polymerization process are aqueous polymer gels (i.e., superabsorbent hydrogels) that are reduced in size to small particles by mechanical forces, then dried using drying procedures and apparatus known in the art. The drying process is followed by pulverization of the resulting superabsorbent material to the desired particle size.

To improve the fluid absorption profile, superabsorbent materials are optimized with respect to one or more of absorption capacity, absorption rate, acquisition time, gel strength and/or permeability. Optimization allows for a reduction in the amount of fluff fiber used in an absorbent article, which results in a thinner article. However, it is very difficult to maximize all of these absorption profile properties simultaneously. Nonetheless, the superabsorbent materials of the present invention maintain the conflicting properties of a high centrifuge retention capacity (CRC) and an excellent permeability, and can provide for an absorbent article with improved fluid intake rate.

In order to use an increased amount of superabsorbent materials and a decreased amount of fluff in the absorbent core 44, it is important to maintain a high liquid permeability. In particular, the permeability of a superabsorbent hydrogel layer formed by swelling in the presence of a fluid is very important to overcome the problem of leakage from the product. A lack of permeability directly impacts the ability of superabsorbent hydrogel layers to acquire and distribute such fluids.

In general, the coating of superabsorbent materials with uncrosslinked polyamines can improve adhesion to cellulosic fluff fibers because of the high flexibility of polyamine molecules. However, low molecular weight, uncrosslinked polyamines can be extracted from the superabsorbent materials by wetting with an aqueous fluid. As a result, the viscosity of the aqueous fluid increases, and the acquisition rate of the superabsorbent materials is reduced. If the polyamine is covalently bound to the superabsorbent materials, the degree of superabsorbent material crosslinking is increased and the absorptive capacity of the material is undesirably reduced. Moreover, covalent bonding of polyamine to the superabsorbent material surface typically occurs at a temperature greater than 150°C, which adversely affects the color of the superabsorbent materials, and ultimately, consumer acceptance of the absorbent article.

In accordance with the present invention, superabsorbent materials having a pH of less than 6 that are coated with a polyamine, and optionally surface crosslinked, are disclosed. The present superabsorbent materials comprise a base polymer having a pH less than 6, and are capable of absorbing several times their weight in water while demonstrating an excellent permeability.

The base polymer can be a homopolymer or a copolymer. The identity of the base polymer is not limited as long as the polymer is an anionic polymer (i.e., contains pendant acid moieties) and is capable of swelling and absorbing at least ten times its weight in water when in a neutralized form. Suitable base polymers are crosslinked polymers having acid groups that are at least partially in the form of a salt, generally an alkali metal or ammonium salt.

As referenced above, the base polymer has a pH of less than 6, such as between 4 and 6, or between 5 and 6, or between 5.5 and 6, measured as discussed below. Suitably, the base polymer has less than about 74 mol% of the pendant acid moieties (e.g., carboxylic acid moieties) present in a neutralized form, such as between about 25 mol% and about 70 mol%, or between about 30 mol% and about 65 mol%, of the pendant acid moieties present in a neutralized form. In accordance with the present invention, the base polymer preferably has a degree of neutralization (DN) between 25 mol% and 74 mol%.

The base polymer of the superabsorbent material of the present invention is a lightly crosslinked polymer capable of absorbing several times its own weight in water and/or saline. Superabsorbent materials can be made by any conventional process for preparing superabsorbent polymers and are well known to those skilled in the art. One process for preparing superabsorbent materials is a solution polymerization method described in U.S. Patent Nos. 4,076,663; 4,286,082; 4,654,039; and 5,145,906. Another process is an inverse suspension polymerization method described in U.S. Patent Nos. 4,340,706; 4,497,930; 4,666,975; 4,507,438; and 4,683,274.

Superabsorbent materials suitable in the present invention may be prepared from one or more monoethylenically unsaturated compounds having at least one acid moiety, such as carboxyl, carboxylic acid anhydride, carboxylic acid salt, sulfonic acid, sulfonic acid salt, sulfuric acid, sulfuric acid salt, phosphoric acid, phosphoric acid salt, phosphonic acid, or phosphonic acid salt. Superabsorbent materials useful in the present invention suitably are prepared from one or more monoethylenically unsaturated, water-soluble carboxyl or carboxylic acid anhydride containing monomer, and

the alkali metal and ammonium salts thereof, wherein these monomers desirably comprise 50 mol% to 99.9 mol% of the base polymer.

The base polymer of the superabsorbent materials of the present invention is suitably a lightly crosslinked acrylic resin, such as lightly crosslinked polyacrylic acid. The lightly crosslinked base polymer can be prepared by polymerizing an acidic monomer containing an acyl moiety, such as acrylic acid, or a moiety capable of providing an acid group (e.g., acrylonitrile) in the presence of an internal crosslinking agent (e.g., a polyfunctional organic compound). The base polymer can contain other copolymerizable units (e.g., other monoethylenically unsaturated comonomers known in the art) as long as the base polymer is at least about 10 mol%, such as at least about 25 mol%, acidic monomer units, such as (meth)acrylic acid. To achieve the full advantage of the present invention, the base polymer preferably contains at least about 50 mol%, such as at least about 75 mol%, or up to about 100 mol%, acidic monomer units. The other copolymerizable units can, for example, help improve the hydrophilicity of the polymer.

Suitable ethylenically unsaturated carboxylic acid and carboxylic acid anhydride monomers useful in the base polymer include acrylic acid, methacrylic acid, ethacrylic acid, α-chloroacrylic acid, α-cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citraconic acid, mesaconic acid, glutaconic acid, aconitic acid, maleic acid, fumaric acid, tricarboxyethylene, and maleic anhydride.

Suitable ethylenically unsaturated sulfonic and phosphonic acid monomers include aliphatic or aromatic vinyl sulfonic acids, such as vinylsulfonic acid, allyl sulfonic acid, vinyl toluene sulfonic acid, styrene sulfonic acid, acrylic and methacrylic sulfonic acids (e.g., sulfoethyl acrylate, sulfoethyl methacrylate, sulfopropyl acrylate, sulfopropyl methacrylate, 2-hydroxy-3-methacryloxypropyl sulfonic acid, and 2-acrylamido-2-methylpropane sulfonic acid); vinylphosphonic acid; allylphosphonic acid; and mixtures thereof.

Exemplary, but nonlimiting, monomers include acrylic acid, methacrylic acid, maleic acid, fumaric acid, maleic anhydride, and the sodium, potassium, and ammonium salts thereof. One particularly suitable monomer is acrylic acid.

The base polymer can also contain additional monoethylenically unsaturated monomers that do not bear a pendant acid group, but are copolymerizable with monomers bearing acid groups. Such compounds include, for example, the amides and nitriles of monoethylenically unsaturated carboxylic acids, including acrylamide, methacrylamide, acrylonitrile, and methacrylonitrile. Examples of other suitable comonomers include, but are not limited to, vinyl esters of saturated $C_{1-4}$ carboxylic acids, such as vinyl formate, vinyl acetate, and vinyl propionate; alkyl vinyl ethers having at least two carbon atoms in the alkyl group, for example, ethyl vinyl ether and butyl vinyl ether; esters of monoethylenically unsaturated $C_{3-18}$ alcohols and acrylic acid, methacrylic acid, or maleic acid; monoesters of maleic acid, for example, methyl hydrogen maleate; acrylic and methacrylic esters of alkoxylated monohydric saturated alcohols, for example, alcohols having 10 to 25 carbon atoms reacted with 2 to 200 moles of ethylene oxide and/or propylene oxide per mole of alcohol; and monoacrylic esters and monomethacrylic esters of polyethylene glycol or polypropylene glycol, the molar masses ($M_n$) of the polyalkylene glycols being up to about 2,000, for example. Further suitable comonomers include, but are not limited to, styrene and alkyl-substituted styrenes, such as ethylstyrene and tert-butylstyrene, and 2-hydroxyethyl acrylate.

Polymerization of the acidic monomers, and any copolymerizable monomers, can be performed by free radical processes in the presence of a polyfunctional organic compound. The base polymers are internally crosslinked to a sufficient extent such that the base polymer is water insoluble. Internal crosslinking renders the base polymer substantially water insoluble, and, in part, serves to determine the absorption capacity of the base polymer. For use in absorption applications, the base polymer is suitably lightly crosslinked, having a crosslinking density of less than about 20%, such as less than about 10%, or between about 0.01% to about 7%.

A crosslinking agent is suitably used in an amount of less than about 7 wt%, such as between about 0.1 wt% and about 5 wt%, based on the total weight of monomers. Examples of crosslinking polyvinyl monomers include, but are not limited to, polyacrylic (or polymethacrylic) acid esters represented by the following formula (I), and bisacrylamides represented by the following formula (II):

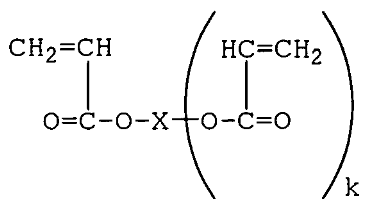

(I)

wherein X is ethylene, propylene, trimethylene, cyclohexyl, hexamethylene, 2-hydroxypropylene, $(CH_2CH_2O)_nCH_2CH_2$, or

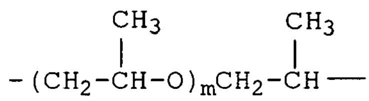

n and m are each an integer 5 to 40, and k is 1 or 2;

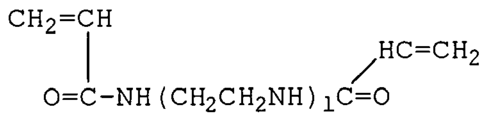

(II)

wherein l is 2 or 3.

The compounds of formula (I) can be prepared by reacting polyols, such as ethylene glycol, propylene glycol, trimethylolpropane, 1,6-hexanediol, glycerin, pentaerythritol, polyethylene glycol, or polypropylene glycol, with acrylic acid or methacrylic acid. The compounds of formula (II) can be obtained by reacting polyalkylene polyamines, such as diethylenetriamine and triethylenetetramine, with acrylic acid.

Specific internal crosslinking agents include, but are not limited to, 1,4-butanediol diacrylate, 1,4-butanediol dimethacrylate, 1,3-butylene glycol diacrylate, 1,3-butylene glycol dimethacrylate, diethylene glycol diacrylate, diethylene glycol dimethacrylate, ethoxylated bisphenol A diacrylate, ethoxylated bisphenol A dimethacrylate, ethylene glycol dimethacrylate, 1,6-hexanediol diacrylate, 1,6-hexanediol dimethacrylate, neopentyl glycol dimethacrylate, polyethylene glycol diacrylate, polyethylene glycol dimethacrylate, triethylene glycol diacrylate, triethylene glycol dimethacrylate, tripropylene glycol diacrylate, tetraethylene glycol diacrylate, tetraethylene glycol dimethacrylate, dipentaerythritol pentaacrylate, pentaerythritol tetraacrylate, pentaerythritol triacrylate, trimethylolpropane triacrylate, trimethylolpropane trimethacrylate, tris(2-hydroxyethyl)-isocyanurate triacrylate, ethoxylated trimethylolpropane triacrylate (ETMPTA) (e.g., ETMPTA ethyoxylated with 15 moles of ethylene oxide (EO) on average), tris(2-hydroxyethyl)isocyanurate trimethyacrylate, divinyl esters of a polycarboxylic acid, diallyl esters of a polycarboxylic acid, triallyl terephthalate, diallyl maleate, diallyl fumarate, hexamethylenebismaleimide, trivinyl trimellitate, divinyl adipate, diallyl succinate, a divinyl ether of ethylene glycol, cyclopentadiene diacrylate, a tetraallyl ammonium halide, divinyl benzene, divinyl ether, diallyl phthalate, or mixtures thereof. Particularly suitable internal crosslinking agents are N,N'-methylenebisacrylamide, N,N'-methylenebismethacrylamide, ethylene glycol dimethacrylate, and trimethylolpropane triacrylate.

The base polymer of the present invention can be any internally crosslinked polymer having pendant acid moieties that acts as a superabsorbent material in its neutralized form. Examples of suitable base polymers include, but are not limited to, polyacrylic acid, hydrolyzed starch-acrylonitrile graft copolymers, starch-acrylic acid graft copolymers, saponified vinyl acetate-acrylic ester copolymers, hydrolyzed acrylonitrile copolymers, hydrolyzed acrylamide copolymers, ethylene-maleic anhydride copolymers, isobutylene-maleic anhydride copolymers, poly(vinylsulfonic acid), poly (vinylphosphonic acid), poly(vinylphosphoric acid), poly(vinylsulfuric acid), sulfonated polystyrene, poly(aspartic acid), poly(lactic acid), and mixtures thereof. The preferred base polymer is a homopolymer or copolymer of acrylic acid or methacrylic acid.

The free radical polymerization can be initiated by an initiator or by electron beams acting on a polymerizable aqueous mixture. Polymerization also can be initiated in the absence of such initiators by the action of high energy radiation in the presence of photoinitiators.

Useful polymerization initiators include, but are not limited to, compounds that decompose into free radicals under polymerization conditions, for example, peroxides, hydroperoxides, persulfates, azo compounds, and redox catalysts. Water-soluble initiators are desirable. In some cases, mixtures of different polymerization initiators are used, for example, mixtures of hydrogen peroxide and sodium peroxodisulfate or potassium peroxodisulfate. Mixtures of hydrogen peroxide and sodium peroxodisulfate can be in any proportion.

Examples of suitable organic peroxides include, but are not limited to, acetylacetone peroxide, methyl ethyl

ketone peroxide, tert-butyl hydroperoxide, cumene hydroperoxide, tert-amyl perpivalate, tert-butyl perpivalate, tert-butyl perneohexanoate, tert-butyl perisobutyrate, tert-butyl per-2-ethylhexanoate, tert-butyl perisononanoate, tert-butyl permaleate, tert-butyl perbenzoate, di(2-ethylhexyl) peroxydicarbonate, dicyclohexyl peroxydicarbonate, di(4-tert-butylcyclohexyl) peroxydicarbonate, dimyristyl peroxydicarbonate, diacetyl peroxydicarbonate, an allyl perester, cumyl peroxyneodecanoate, tert-butyl per-3,5,5-trimethylhexanoate, acetylcyclohexylsulfonyl peroxide, dilauryl peroxide, dibenzoyl peroxide, and tert-amyl perneodecanoate. Particularly suitable polymerization initiators are water-soluble azo initiators, for example 2,2'-azobis(2-amidinopropane) dihydrochloride, 2,2'-azobis(N,N'-dimethylene)isobutyramidine dihydrochloride, 2-(carbamoylazo-isobutyronitrile, 2,2'-azobis[2-(2'-imidazolin-2-yl)propane] dihydrochloride, and 4,4'-azobis(4-cyanovaleric acid). The polymerization initiators are used, for example, in amounts of between about 0.01 % to about 5%, such as about 0.05% to about 2.0% by weight, based on the monomers to be polymerized.

Suitable polymerization initiators also include redox catalysts. In redox catalysts, the oxidizing compound comprises at least one of the above-specified per compounds, and the reducing component comprises, for example, ascorbic acid, glucose, sorbose, ammonium or alkali metal bisulfite, sulfite, thiosulfate, hyposulfite, pyrosulfite, or sulfide, or a metal salt, such as iron (II) ions or sodium hydroxymethylsulfoxylate. The reducing component of the redox catalyst preferably is ascorbic acid or sodium sulfite. Based on the amount of monomers used in the polymerization, about 3 x $10^{-6}$ to about 1 mol% of the reducing component of the redox catalyst system can be used, and about 0.001 to about 5.0 mol% of the oxidizing component of the redox catalyst can be used, for example.

When polymerization is initiated using high energy radiation, the initiator typically comprises a photoinitiator. Photoinitiators include, for example, $\alpha$-splitters, H-abstracting systems, and azides. Examples of such initiators include, but are not limited to, benzophenone derivatives, such as Michler's ketone; phenanthrene derivatives; fluorene derivatives; anthraquinone derivatives; thioxanthone derivatives; coumarin derivatives; benzoin ethers and derivatives thereof; azo compounds, such as the above-mentioned free-radical formers, substituted hexaarylbisimidazoles, acylphosphine oxides; or mixtures thereof.

Examples of azides include, but are not limited to, 2-(N,N-dimethylamino)ethyl 4-azidocinnamate, 2-(N,N-dimethylamino)ethyl 4-azidonaphthyl ketone, 2-(N,N-dimethylamino)ethyl 4-azidobenzoate, 5-azido-1-naphthyl 2'-(N,N-dimethylamino)ethyl sulfone, N-(4-sulfonylazidophenyl)maleimide, N-acetyl-4-sulfonylazidoaniline, 4-sulfonyl-azidoaniline, 4-azidoaniline, 4-azidophenacyl bromide, p-azidobenzoic acid, 2,6-bis(p-azidobenzylidene)cyclohexanone, and 2,6-bis(p-azidobenzylidene)-4-methylcyclohexanone. Photoinitiators can be used, if at all, in amounts of about 0.01 % to about 5% by weight of the monomers to be polymerized.

The base polymer of the present invention is partially neutralized. As previously stated, the degree of neutralization is suitably between about 25 mol% and about 70 mol%, such as between about 30 mol% and about 65 mol%, or about 35 mol% to about 60 mol%, based on monomers containing acid groups. In accordance with the present invention, the base polymer is neutralized to a sufficient degree such that the pH of the superabsorbent hydrogel is less than 6, such as less than about 5.8. To achieve the full advantage of the present invention, the pH of the superabsorbent hydrogel is about 5 to about 5.7.

In accordance with the present invention, if the pH of the base polymer particle is below 4, over-curing can occur between the polyamine and the pendant carboxylic acid groups of the superabsorbent materials during heating, which leads to a decreased CRC and an increased gel bed permeability (GBP). When a base polymer particle has a pH of 6 or above, the GBP is adversely affected.

Useful neutralizing agents for the base polymer include alkali metal bases, ammonia, and/or amines. Preferably, the neutralizing agent comprises aqueous sodium hydroxide, aqueous potassium hydroxide, or lithium hydroxide. However, neutralization also can be achieved using sodium carbonate, sodium bicarbonate, potassium carbonate, or potassium bicarbonate, or other carbonates or bicarbonates, as a solid or as a solution. Primary, secondary and/or tertiary amines can be used to neutralize the base polymer.

Neutralization of the base polymer can be performed before, during, or after the polymerization in a suitable apparatus for this purpose. For example, the neutralization can be performed directly in a kneader used for polymerization of the monomers. The varying degree of neutralization to achieve a pH value less than about 6 is related to the chemical identity of the base polymer.

In accordance with the present invention, polymerization of an aqueous monomer solution (e.g., gel polymerization) is desirable. In this method, a 10% to 70% by weight aqueous solution of the monomers, including the internal crosslinking agent, is neutralized in the presence of a free radical initiator. The solution polymerization is suitably performed at about 0°C to about 150°C, such as about 10°C to about 100°C, and at atmospheric, superatmospheric or reduced pressure. The polymerization also can be conducted under a protective gas atmosphere, suitably under nitrogen.

After polymerization, the resulting hydrogel of the base polymer is dried, and the dry base polymer materials are ground and classified. The base polymer materials typically are surface crosslinked. In accordance with the present invention, surface crosslinking is optional. In one embodiment, the base polymer particles can be surface crosslinked, then coated with a polyamine. Suitably, surface crosslinking is performed simultaneously with applying a polyamine coating on the base polymer particles.

In one aspect of applying a polyamine coating to the base polymer particles, a coating solution containing a polyamine dissolved in a solvent can be applied to the surfaces of the base polymer particles. Then, optional coating solution(s) containing an optional surface crosslinking agent and/or an optional inorganic salt having a polyvalent metal cation, dissolved or dispersed in a suitable solvent, can be applied to the surfaces of the superabsorbent materials. Then, the coated base polymer particles are heated for a sufficient time at a sufficient temperature to evaporate the solvents of the coating solutions, to surface crosslink the base polymer particles (if an optional surface crosslinking agent is used), and to form a polyamine coating on the base polymer particles to provide superabsorbent materials of the present invention.

It should be understood that the order of applying the polyamine, optional surface crosslinking agent, and optional inorganic salt to the surfaces of the base polymer particles, is not critical. The components can be added in any order, either from two or three solutions. However, the polyamine and optional inorganic salt should be applied from different solutions to avoid an interaction prior to application to the base polymer particles.

In other aspects, the base polymer particles can be surface crosslinked prior to application of the polyamine and optional inorganic salt. In still other aspects, a surface crosslinking agent can be applied to the base polymer particles, followed by the polyamine and optional inorganic salt, and the particles are then heated to form surface crosslinks and the polyamine coating simultaneously.

In the optional surface crosslinking process, a multifunctional compound capable of reacting with the functional groups of the base polymer can be applied to the surface of the base polymer particles, preferably using an aqueous solution. The aqueous solution can also contain water-miscible organic solvents, such as an alcohol (e.g., such as methanol, ethanol, or i-propanol); a polyol, such as ethylene glycol or propylene glycol; or acetone.

A solution of a surface crosslinking agent is applied to the base polymer particles in an amount to wet predominantly only the outer surfaces of the base polymer particles, either before or after application of the polyamine. Surface crosslinking and drying of the base polymer particles can then be performed, suitably by heating at least the wetted surfaces of the base polymer particles.

Typically, the base polymer particles are surface treated with a solution of a surface crosslinking agent containing about 0.01% to about 4% by weight surface crosslinking agent, such as about 0.4% to about 2% by weight surface crosslinking agent in a suitable solvent. The solution can be applied as a fine spray onto the surfaces of freely tumbling base polymer particles at a ratio of about 1:0.01 to about 1:0.5 parts by weight base polymer particles to solution of surface crosslinking agent. The surface crosslinking agent, if present at all, can be present in an amount of about 0.001 % to about 5% by weight of the base polymer particles, such as about 0.001 % to about 0.5% by weight. To achieve the full advantage of the present invention, the surface crosslinking agent is present in an amount of about 0.001 % to about 0.1 % by weight of the base polymer particles.

Surface crosslinking and drying of the base polymer particles are achieved by heating the surface-treated base polymer particles at a suitable temperature, such as between about 70°C to about 150°C, or between about 105°C to about 120°C. Suitable surface crosslinking agents are capable of reacting with acid moieties and crosslinking polymers at the surfaces of the base polymer particles.

Nonlimiting examples of suitable surface crosslinking agents include, but are not limited to, an alkylene carbonate, such as ethylene carbonate or propylene carbonate; a polyaziridine, such as 2,2-bishydroxymethyl butanol tris [3-(1-aziridine propionate] or bis-N-aziridinomethane; a haloepoxy, such as epichlorohydrin; a polyisocyanate, such as 2,4-toluene diisocyanate; a di- or polyglycidyl compound, such as diglycidyl phosphonates, ethylene glycol diglycidyl ether, or bischlorohydrin ethers of polyalkylene glycols; alkoxysilyl compounds; polyols such as ethylene glycol, 1,2-propanediol, 1,4-butanediol, glycerol, methyltriglycol, polyethylene glycols having an average molecular weight ($M_w$) of 200-10,000, di- and polyglycerol, pentaerythritol, sorbitol, the ethoxylates of these polyols and their esters with carboxylic acids or carbonic acid, such as ethylene carbonate or propylene carbonate; carbonic acid derivatives, such as urea, thiourea, guanidine, dicyandiamide, 2-oxazolidinone and its derivatives, bisoxazoline, polyoxazolines, di- and polyisocyanates; di- and poly-N-methylol compounds, such as methylenebis(N-methylolmethacrylamide) or melamine-formaldehyde resins; compounds having two or more blocked isocyanate groups, such as trimethylhexamethylene diisocyanate blocked with 2,2,3,6-tetramethylpiperidin-4-one; and other surface crosslinking agents known to persons skilled in the art.

A solution of the optional surface crosslinking agent can be applied to the surfaces of the base polymer particles before or after a solution containing the polyamine is applied to the surfaces of the base polymer particles. The polyamine also can be applied to the base polymer particles after the surface crosslinking step has been completed.

A solution containing the polyamine can comprise about 5% to about 50% by weight of a polyamine in a suitable solvent. Suitably, a sufficient amount of a solvent is present to allow the polyamine to be readily and homogeneously applied to the surfaces of the base polymer particles. The solvent for the polyamine solution can be, but is not limited to, water, an alcohol, or a glycol, such as methanol, ethanol, ethylene glycol, or propylene glycol, and mixtures thereof.

The amount of polyamine applied to the surfaces of the base polymer particles is suitably sufficient to coat the base polymer particle surfaces. Accordingly, the amount of polyamine applied to the surfaces of the base polymer particles is preferably about 0.1 % to about 2%, such as about 0.2% to about 1 %, of the weight of the base polymer

particle. To achieve the full advantage of the present invention, the polyamine is present on the base polymer particle surfaces in an amount of about 0.2% to about 0.5% by weight of the base polymer particle.

A polyamine forms an ionic bond with a base polymer and retains adhesive forces to the base polymer after the base polymer absorbs a fluid and swells. Desirably, an excessive amount of covalent bonds are not formed between the polyamine and the base polymer, and the polyamine-base polymer interactions are intermolecular, such as electrostatic, hydrogen bonding, and van der Waals interactions. Therefore, the presence of a polyamine on the base polymer particles does not adversely influence the absorption profile of the base polymer particles.

A polyamine useful in the present invention has at least two, and preferably a plurality of, nitrogen atoms per molecule. The polyamine typically has a weight average molecular weight ($M_w$) of about 5,000 to about 1,000,000, such as about 20,000 to about 300,000. To achieve the full advantage of the present invention, the polyamine has an $M_w$ of about 100,000 to about 300,000.

In general, useful polyamine polymers have primary amine groups, secondary amine groups, tertiary amine groups, quaternary ammonium groups, or mixtures thereof. Examples of polyamines include, but are not limited to, a polyvinylamine, a polyallylamine, a polyethyleneimine, a polyalkyleneamine, a polyazetidine, a polyvinylguanidine, a poly(DADMAC) (i.e., a poly(diallyl dimethyl ammonium chloride), a cationic polyacrylamide, a polyamine functionalized polyacrylate, and mixtures thereof.

Homopolymers and copolymers of vinylamine also can be used, such as copolymers of vinylformamide and comonomers for example, which are converted to vinylamine copolymers. The comonomers can be any monomer capable of copolymerizing with vinylformamide. Nonlimiting examples of such monomers include, but are not limited to, acrylamide, methacrylamide, methacrylonitrile, vinylacetate, vinylpropionate, styrene, ethylene, propylene, N-vinylpyrrolidone, N-vinylcaprolactam, N-vinylimidazole, monomers containing a sulfonate or phosphonate group, vinylglycol, acrylamido(methacrylamido)alkylene trialkyl ammonium salt, diallyl dialkylammonium salt, $C_{1-4}$alkyl vinyl ethers such as methyl vinyl ether, ethyl vinyl ether, isopropyl vinyl ether, n-propyl vinyl ether, t-butyl vinyl ether, N-substituted alkyl (meth)acrylamides substituted by a $C_{1-4}$alkyl group as, for example, N-methylacrylamide, N-isopropylacrylamide, and N,N-dimethylacrylamide, $C_{1-20}$alkyl(meth)acrylic acid esters such as methyl methacrylate, ethyl methacrylate, propyl acrylate, butyl acrylate, hydroxyethyl acrylate, hydroxyethyl methacrylate, hydroxypropyl acrylate, hydroxypropyl methacrylate, hydroxybutyl acrylate, hydroxybutyl methacrylate, 2-methylbutyl acrylate, 3-methylbutyl acrylate, 3-pentyl acrylate, neopentyl acrylate, 2-methylpentyl acrylate, hexyl acrylate, cyclohexyl acrylate, 2-ethylhexyl acrylate, phenyl acrylate, heptyl acrylate, benzyl acrylate, tolyl acrylate, octyl acrylate, 2-octyl acrylate, nonyl acrylate, and octyl methacrylate.

Specific copolymers of polyvinylamine include, but are not limited to, copolymers of N-vinylformamide and vinyl acetate, vinyl propionate, a $C_{1-4}$alkyl vinyl ether, a (meth)acrylic acid ester, acrylonitrile, acrylamide and vinylpyrrolidone.

In accordance with the present invention, the number of covalent bonds that form between the polyamine and the base polymer is extremely low, if present at all. However, for some polyamines, a polyamine coating can impart a tack to the base polymer particles, which could lead to agglomeration or aggregation of coated base polymer particles. Therefore, a second coating solution which contains an optional inorganic salt having a polyvalent cation (i.e., a cation having a valence of two, three, or four) can be applied to the surfaces of the base polymer.

The polyvalent metal cation is capable of interacting (e.g., forming ionic crosslinks) with the nitrogen atoms of the polyamine. In addition, the polyamine can interact (e.g., form ionic links) with the base polymer because of the low pH of the base polymer particles. As a result, a monolithic, and tackless, polyamine coating can be formed on the surface of the base polymer to provide coated superabsorbent materials of the present invention.

In accordance with the present invention, an optional inorganic salt applied to surfaces of the base polymer particles has a sufficient water solubility such that polyvalent metal cations are available to interact with the nitrogen atoms of the polyamine. Accordingly, a useful inorganic salt has a water solubility of at least about 0.1 grams (g) of inorganic salt per 100 milliliters (ml) of water, such as at least about 0.2 g per 100 ml of water.

The polyvalent metal cation of the optional inorganic salt has a valence of +2, +3, or +4 (i.e., in the range of +2 to +4) and can be, but is not limited to, $Mg^{2+}$, $Ca^{2+}$, $Al^{3+}$ $Sc^{3+}$, $Ti^{4+}$, $Mn^{2+}$, $Fe^{2+}$, $Fe^{3+}$, $Co^{2+}$, $Ni^{2+}$, $Cu^{+/2+}$, $Zn^{2+}$, $Y^{3+}$, $Zr^{4+}$, $La^{3+}$, $Ce^{4+}$, $Hf^{4+}$, $Au^{3+}$, and mixtures thereof. Desirable cations are $Mg^{2+}$, $Ca^{2+}$, $Al^{3+}$, $Ti^{4+}$, $Zr^{4+}$, $La^{3+}$, and mixtures thereof, and particularly desirable cations are $Al^{3+}$, $Ti^{4+}$, $Zr^{4+}$, and mixtures thereof. The anion of the inorganic salt is not limited, as long as the inorganic salt has sufficient solubility in water. Examples of anions include, but are not limited to, chloride, bromide, nitrate and sulfate.

The optional inorganic salt often can be present in a coating solution together with the optional surface crosslinking agent. The optional inorganic salt typically is present in a coating solution in an amount of about 0.5% to about 20% by weight, for example. The amount of optional inorganic salt present in a coating solution, and the amount applied to the base polymer particles, is related to the identity of the inorganic salt, its solubility in the solvent of the coating solution, the identity of the polyamine applied to the base polymer particles, and the amount of polyamine applied to the base polymer particles. In general, the amount of optional inorganic salt applied to the base polymer particles is sufficient to form a tackless, monolithic polyamine coating.

In accordance with the present invention, the polyamine and optional inorganic salt are applied to the base

polymer particles in a manner such that each is uniformly distributed on the surfaces of the base polymer particles. Any known method for applying a liquid to a solid can be used, preferably by dispersing a coating solution into fine droplets, for example, by use of a pressurized nozzle or a rotating disc. Uniform coating of the base polymer particles can be achieved in a high intensity mechanical mixer or a fluidized mixer, which suspends the base polymer particles in a turbulent gas stream. Methods for the dispersion of a liquid onto the surfaces of base polymer particles are known in the art, such as that described in U.S. Patent No. 4,734,478.

Methods of coating the base polymer particles can include applying the polyamine and optional inorganic salt simultaneously. The two components are suitably applied via two separate nozzles to avoid interacting before application to the surfaces of the base polymer particles. One suitable method of coating the base polymer is a sequential addition of the components. A more suitable method is application of the polyamine first, followed by an application of the optional inorganic salt. The resulting coated base polymer particles are then heated between about 70°C and about 175°C for sufficient time (e.g., about 5 to about 90 minutes) to cure the polyamine coating.

As referenced above, the absorbent core 44 can also optionally include fluff, such as cellulosic fibers. Such cellulosic fibers may include, but are not limited to, chemical wood pulps such as sulfite and sulfate (sometimes called Kraft) pulps, as well as mechanical pulps such as ground wood, thermomechanical pulp and chemithermomechanical pulp. More particularly, the pulp fibers may include cotton, other typical wood pulps, cellulose acetate, debonded chemical wood pulp, and combinations thereof. Pulps derived from both deciduous and coniferous trees can be used. Additionally, the cellulosic fibers may include such hydrophilic materials as natural plant fibers, milkweed floss, cotton fibers, microcrystalline cellulose, microfibrillated cellulose, or any of these materials in combination with wood pulp fibers. Suitable cellulosic fluff fibers can include, for example, NB480 (available from Weyerhaeuser Co., a business having offices located in Federal Way, Washington U.S.A.); NB416, a bleached southern softwood Kraft pulp (available from Weyerhaeuser Co.); CR 54, a bleached southern softwood Kraft pulp (available from Bowater Inc., a business having offices located in Greenville, South Carolina U.S.A.).; SULPHATATE HJ, a chemically modified hardwood pulp (available from Rayonier Inc., a business having offices located in Jesup, Georgia U.S.A.); NF 405, a chemically treated bleached southern softwood Kraft pulp (available from Weyerhaeuser Co.); and CR 1654, a mixed bleached southern softwood and hardwood Kraft pulp (available from Bowater Inc.)

The absorbent core 44 can be formed using methods known in the art. While not being limited to the specific method of manufacture, the absorbent core can utilize a meltblown process and can further be formed on a coform line. Exemplary meltblown processes are described in various patents and publications, including NRL Report 4364, "Manufacture of Super-Fine Organic Fibers" by V. A. Wendt, E. L. Boone and C. D. Fluharty; NRL Report 5265, "An Improved Device For the Formation of Super-Fine Thermoplastic Fibers" by K. D. Lawrence, R. T. Lukas and J. A. Young; and U.S. Patent Nos. 3,849,241 and 5,350,624.

To form "coform" materials, additional components are mixed with the meltblown fibers as the fibers are deposited onto a forming surface. For example, the superabsorbent materials of the present invention and fluff such as wood pulp fibers may be injected into the meltblown fiber stream so as to be entrapped and/or bonded to the meltblown fibers. Exemplary coform processes are described in U.S. Patent Nos. 4,100,324 to Anderson et al.; 4,587,154 to Hotchkiss et al.; 4,604,313 to McFarland et al.; 4,655,757 to McFarland et al.; 4,724,114 to McFarland et al.; 4,100,324 to Anderson et al.; and U.K. Patent GB 2,151,272 to Minto et al. Absorbent, elastomeric meltblown webs containing high amounts of superabsorbent are described in U.S. Patent No. 6,362,389 to D. J. McDowall, and absorbent, elastomeric meltblown webs containing high amounts of superabsorbent and low superabsorbent shake-out values are described in pending U.S. Patent Application Publication No. 20060004336 (Serial No. 10/883174) to X. Zhang et al.

One example of a method of forming the absorbent core 44 of the present invention is illustrated in FIG 3. The dimensions of the apparatus in FIG 3 are described herein by way of example. Other types of apparatus having different dimensions and/or different structures may also be used to form the absorbent core 44. As shown in FIG 3, elastomeric material 72 in the form of pellets can be fed through two pellet hoppers 74 into two single screw extruders 76 that each feed a spin pump 78. The elastomeric material 72 may be a multicomponent elastomer blend available under the trade designation KRATON G2755 from Kraton Polymers, as well as others mentioned above. Each spin pump 78 feeds the elastomeric material 72 to a separate meltblown die 80. Each meltblown die 80 may have 30 holes per 2.5cm (inch, hpi). The die angle may be adjusted anywhere between 0 and 70 degrees from horizontal, and is suitably set at about 45 degrees. The forming height may be at a maximum of about 40.6cm (16 inches), but this restriction may differ with different equipment.

A chute 82 having a width of about 61 cm (24 inches) may be positioned between the meltblown dies 80. The depth, or thickness, of the chute 82 may be adjustable in a range from about 1.3 to about 3.18cm (0.5 to about 1.25 inches), or from about 1.9 to about 2.5cm (0.75 to about 1.0 inch). A picker 144 connects to the top of the chute 82. The picker 144 is used to fiberize the pulp fibers 86. The picker 144 may be limited to processing low strength or debonded (treated) pulps, in which case the picker 144 may limit the illustrated method to a very small range of pulp types. In contrast to conventional hammermills that use hammers to impact the pulp fibers repeatedly, the picker 144 uses small teeth to tear the pulp fibers 86 apart. Suitable pulp fibers 86 for use in the method illustrated in FIG 3 include those

mentioned above, such as NB480.

At an end of the chute 82 opposite the picker 144 is a superabsorbent material feeder 88. The feeder 88 pours the superabsorbent material 90 of the present invention into a hole 92 in a pipe 94 which then feeds into a blower fan 96. Past the blower fan 96 is a length of 10.2cm (4-inch) diameter pipe 98 sufficient for developing a fully developed turbulent flow at about 1524m (5,000 feet) per minute, which allows the superabsorbent material 90 to become distributed. The pipe 98 widens from a 10.2cm (4-inch) diameter to the 61 cm (24-inch) by 1.9cm (0.75-inch) chute 82, at which point the superabsorbent material 90 mixes with the pulp fibers 86 and the mixture falls straight down and gets mixed on either side at an approximately 45-degree angle with the elastomeric material 72. The mixture of superabsorbent material 90, pulp fibers 86, and elastomeric material 72 falls onto a wire conveyor 100 moving from about 1.2m (14 feet) to about 10.7m (35 feet) per minute. However, before hitting the wire conveyor 100, a spray boom 102 optionally sprays an aqueous surfactant mixture 104 in a mist through the mixture, thereby rendering the resulting absorbent core 44 wettable. The surfactant mixture 104 may be a 1:3 mixture of GLUCOPON 220 UP and AHCOVEL Base N-62, available from Cognis Corp. and Uniqema, respectively. An under wire vacuum 106 is positioned beneath the conveyor 100 to assist in forming the absorbent core 44.

In general, the absorbent core 44 is typically a unitary structure comprising a substantially uniform distribution of superabsorbent materials, fibers, and any other optional additives. However, referring to FIG 4, it has been discovered that the absorbent core 44 of the present invention may be further enhanced through structural modifications when combined with superabsorbent materials of the present invention. For example, providing a layer 60 comprising substantially superabsorbent materials of the present invention sandwiched between layers 62 and 64 comprising substantially fluff fibers, such as NB480, can result in an absorbent core 44 having improved absorbent properties, such as fluid insult intake rate, when compared to a structure comprising a substantially uniform distribution of the superabsorbent materials and fluff fibers. Such layering can occur in the z-direction of the absorbent core 44 and may optionally cover the entire x-y area. However, the layers 60 through 64 need not be discreet from one another. For example, in some aspects, the z-directional middle portion 60 of the absorbent core need only contain a higher superabsorbent material percentage (e.g., at least about 10% by weight higher) than the top layer 62 and/or bottom layer 64 of the absorbent core 44. Desirably, the layers 60 through 64 are present in the area of the absorbent core 44 that is within an insult target zone.

The present invention may be better understood with reference to the following examples.

## TEST PROCEDURES

### p H Test

One hundred milliliters (100 ml) of a 0.9% by weight sodium chloride (NaCl) solution are magnetically stirred at moderate speed in a 150 ml beaker without air being drawn into the solution. This solution is admixed with 0.5 ± 0.001 grams of the superabsorbent materials to be tested, and the resulting mixture is stirred for 10 minutes. After 10 minutes, the pH of the mixture is measured with a pH glass electrode, and the value is recorded only after it is stable, and, at the earliest, after 1 minute.

### Centrifuge Retention Capacity (CRC) Test

This test determines the free swelling capacity of a hydrogel-forming polymer. In this method, 0.2000 ± 0.0050 grams of dry superabsorbent material of size fraction 106 to 850 μm are inserted into a teabag. The teabag is placed in saline solution (i.e., 0.9 wt% aqueous sodium chloride) for 30 minutes (at least 0.83 l (liter) saline solution/1 g polymer). Then, the teabag is centrifuged for 3 minutes at 250 G. The absorbed quantity of saline solution is determined by measuring the weight of the teabag.

### Free-Swell Gel Bed Permeability (GBP) Test

This procedure is disclosed in U.S. Patent No. 6,387,495. The results are expressed in Darcies.

### Absorbency Under Load (AUL) Test

This procedure is disclosed in EP-A 0615736. The results are expressed in g/g.

### 0.3 psi (2.1 kPa) Gel Bed Permeability (GBP 0.3 psi) Test

This procedure is disclosed in pending U.S. Patent Application, publication No. 20050027268 (Serial No.

10/631916). The results are expressed in Darcies.

**Saturated Capacity Test**

Saturated Capacity is determined using a Saturated Capacity (SAT CAP) tester with a Magnahelic vacuum gage and a latex dam, comparable to the following description. Referring to FIGs 5-7, a Saturated Capacity tester vacuum apparatus 110 comprises a vacuum chamber 112 supported on four leg members 114. The vacuum chamber 112 includes a front wall member 116, a rear wall member 118, and two side walls 120 and 121. The wall members are sufficiently thick to withstand the anticipated vacuum pressures, and are constructed and arranged to provide a chamber having outside dimensions measuring 59.7cm (23.5 inches) in length, 35.6cm (14 inches) in width and 20.3cm (8 inches) in depth.

A vacuum pump (not shown) operably connects with the vacuum chamber 112 through an appropriate vacuum line conduit and a vacuum valve 124. In addition, a suitable air bleed line connects into the vacuum chamber 112 through an air bleed valve 126. A hanger assembly 128 is suitably mounted on the rear wall 118 and is configured with S-curved ends to provide a convenient resting place for supporting a latex dam sheet 130 in a convenient position away from the top of the vacuum apparatus 110. A suitable hanger assembly can be constructed from 6.4mm (0.25 inch) diameter stainless steel rod. The latex dam sheet 130 is looped around a dowel member 132 to facilitate grasping and to allow a convenient movement and positioning of the latex dam sheet 130. In the illustrated position, the dowel member 132 is shown supported in a hanger assembly 128 to position the latex dam sheet 130 in an open position away from the top of the vacuum chamber 112.

A bottom edge of the latex dam sheet 130 is clamped against a rear edge support member 134 with suitable securing means, such as toggle clamps 140. The toggle clamps 140 are mounted on the rear wall member 118 with suitable spacers 141 which provide an appropriate orientation and alignment of the toggle clamps 140 for the desired operation. Three support shafts 142 are 1.9cm (0.75 inches) in diameter and are removably mounted within the vacuum chamber 112 by means of support brackets 144. The support brackets 144 are generally equally spaced along the front wall member 116 and the rear wall member 118 and arranged in cooperating pairs. In addition, the support brackets 144 are constructed and arranged to suitably position the uppermost portions of the support shafts 142 flush with the top of the front, rear and side wall members of the vacuum chamber 112. Thus, the support shafts 142 are positioned substantially parallel with one another and are generally aligned with the side wall members 120 and 121. In addition to the rear edge support member 134, the vacuum apparatus 110 includes a front support member 136 and two side support members 138 and 139. Each side support member measures about 2.5cm (1 inch) in width and about 3.2cm (1.25 inches) in height. The lengths of the support members are constructed to suitably surround the periphery of the open top edges of the vacuum chamber 112, and are positioned to protrude above the top edges of the chamber wall members by a distance of about 1.3cm (0.5 inches).

A layer of egg crating type material 146 is positioned on top of the support shafts 142 and the top edges of the wall members of the vacuum chamber 112. The egg crate material extends over a generally rectangular area measuring 59.7cm (23.5 inches) by 35.6cm (14 inches), and has a depth measurement of about 9.7mm (0.38 inches). The individual cells of the egg crating structure measure about 1.3cm (0.5 inch) square, and the thin sheet material comprising the egg crating is composed of a suitable material, such as polystyrene. For example, the egg crating material can be McMaster-Carr Supply Catalog No. 162 4K 14 (available from McMaster-Carr Supply Company, a business having offices located in Atlanta, Georgia U.S.A.) translucent diffuser panel material. A layer of 6 mm (0.25 inch) mesh TEFLON-coated screening 148 (available from Eagle Supply and Plastics, Inc., a business having offices located in Appleton, Wisconsin, U.S.A.) which measures 59.7cm (23.5 inches) by 35.6cm (14 inches), is placed on top of the egg crating material 146.

A suitable drain line and a drain valve 150 connect to the bottom plate member 119 of the vacuum chamber 112 to provide a convenient mechanism for draining liquids from the vacuum chamber 112. The various wall members and support members of the vacuum apparatus 110 may be composed of a suitable non-corroding, moisture-resistant material, such as polycarbonate plastic. The various assembly joints may be affixed by solvent welding and/or fasteners, and the finished assembly of the tester is constructed to be water-tight. A vacuum gauge 152 operably connects through a conduit into the vacuum chamber 112. A suitable pressure gauge is a Magnahelic differential gauge capable of measuring a vacuum of 0-254cm (0-100 inches) of water, such as a No. 2100 gauge available from Dwyer Instrument Incorporated (a business having offices located in Michigan City, Indiana, U.S.A.)

The dry product or other absorbent structure is weighed and then placed in excess 0.9% NaCl saline solution, submerged and allowed to soak for twenty (20) minutes. After the twenty (20) minute soak time, the absorbent structure is placed on the egg crate material and mesh TEFLON-coated screening of the Saturated Capacity tester vacuum apparatus 110. The latex dam sheet 130 is placed over the absorbent structure(s) and the entire egg crate grid so that the latex dam sheet 130 creates a seal when a vacuum is drawn on the vacuum apparatus 110. A vacuum of 3.4kPa (0.5 pounds per square inch (psi)) is held in the Saturated Capacity tester vacuum apparatus 110 for five minutes. The vacuum creates a pressure on the absorbent structure(s), causing drainage of some liquid. After five minutes at 3.4kPa

(0.5 psi) vacuum, the latex dam sheet 130 is rolled back and the absorbent structure(s) are weighed to generate a wet weight.

The overall capacity of each absorbent structure is determined by subtracting the dry weight of each absorbent from the wet weight of that absorbent, determined at this point in the procedure. The 3.4kPa (0.5 psi) Saturated Capacity or Saturated Capacity of the absorbent structure is determined by the following formula:

$$\text{Saturated Capacity} = (\text{wet weight} - \text{dry weight}) / \text{dry weight};$$

wherein the Saturated Capacity value has units of grams of fluid/gram of absorbent. For Saturated Capacity, a minimum of three specimens of each sample should be tested and the results averaged. If the absorbent structure has low integrity or disintegrates during the soak or transfer procedures, the absorbent structure can be wrapped in a containment material such as paper towelling, for example SCOTT paper towels manufactured by Kimberly-Clark Corporation, a business having offices located in Neenah, Wisconsin, U.S.A. The absorbent structure can be tested with the overwrap in place and the capacity of the overwrap can be independently determined and subtracted from the wet weight of the total wrapped absorbent structure to obtain the wet absorbent weight.

**Fluid Intake Rate Test**

The Fluid Intake Rate (FIR) Test determines the amount of time required for an absorbent structure to take in (but not necessarily absorb) a known amount of test solution (0.9 weight percent solution of sodium chloride in distilled water at room temperature). A suitable apparatus for performing the FIR Test is shown in FIGs 8 and 9 and is generally indicated at 200. The test apparatus 200 comprises upper and lower assemblies, generally indicated at 202 and 204 respectively, wherein the lower assembly comprises a generally 17.8cm by 17.8 cm (7 inch by 7 inch) square lower plate 206 constructed of a transparent material such as PLEXIGLAS (available from Degussa AG, a business having offices located in Dusseldorf, Germany) for supporting the absorbent sample during the test and a generally 11.4cm by 11.4cm (4.5 inch by 4.5 inch) square platform 218 centered on the lower plate 206.

The upper assembly 202 comprises a generally square upper plate 208 constructed similar to the lower plate 206 and having a central opening 210 formed therein. A cylinder (fluid delivery tube) 212 having an inner diameter of about 2.5cm (one inch) is secured to the upper plate 208 at the central opening 210 and extends upward substantially perpendicular to the upper plate. The central opening 210 of the upper plate 208 should have a diameter at least equal to the inner diameter of the cylinder 212 where the cylinder 212 is mounted on top of the upper plate 208. However, the diameter of the central opening 210 may instead be sized large enough to receive the outer diameter of the cylinder 212 within the opening so that the cylinder 212 is secured to the upper plate 208 within the central opening 210.

Pin elements 214 are located near the outside corners of the lower plate 206, and corresponding recesses 216 in the upper plate 208 are sized to receive the pin elements 214 to properly align and position the upper assembly 202 on the lower assembly 204 during testing. The weight of the upper assembly 202 (e.g., the upper plate 208 and cylinder 212) is approximately 360 grams to simulate approximately 0.76kPa (0.11 pounds/square inch(psi)) pressure on the absorbent sample during the FIR Test.

To run the FIR Test, an absorbent sample 207 being 7.6cm (three inches) in diameter is weighed and the weight is recorded in grams. The sample 207 is then centered on the platform 218 of the lower assembly 204. The upper assembly 202 is placed over the sample 207 in opposed relationship with the lower assembly 204, with the pin elements 214 of the lower plate 206 seated in the recesses 216 formed in the upper plate 208 and the cylinder 212 is generally centered over the sample 207. Prior to running the FIR test, the aforementioned Saturated Capacity Test is measured on the sample 207. Thirty percent (30%) of the saturation capacity is then calculated by multiplying the mass of the dry sample (grams) times the measured saturated capacity (gram/gram) times 0.3; e.g., if the test sample has a saturated capacity of 20g of 0.9% NaCl saline test solution/g of test sample and the three inch diameter sample 207 weighs one gram, then 6 grams of 0.9% NaCl saline test solution (referred to herein as a first insult) is poured into the top of the cylinder 212 and allowed to flow down into the absorbent sample 207. A stopwatch is started when the first drop of solution contacts the sample 207 and is stopped when the liquid ring between the edge of the cylinder 212 and the sample 207 disappears. The reading on the stopwatch is recorded to two decimal places and represents the intake time (in seconds) required for the first insult to be taken into the absorbent sample 207.

A time period of fifteen minutes is allowed to elapse, after which a second insult equal to the first insult is poured into the top of the cylinder 212 and again the intake time is measured as described above. After fifteen minutes, the procedure is repeated for a third insult. An intake rate (in milliliters/second) for each of the three insults is determined by dividing the amount of solution (e.g., six grams) used for each insult by the intake time measured for the corresponding insult.

At least three samples of each absorbent test is subjected to the FIR Test and the results are averaged to determine the intake rate.

## EXAMPLES

For the examples described below, superabsorbent particles were coated using the following procedure, unless otherwise stated. A quantity of base polymer particles was placed into a standard food processor. The food processor was then turned on and a first coating solution was injected into the food processor using a syringe and was allowed to coat the particles. In some instances, following the first solution, a second solution was injected into the food processor using a syringe and was also allowed to coat the particles. The food processor was then turned off and the superabsorbent materials were removed.

### Example 1

In this example, 40 grams of sodium polyacrylate base polymer having a Centrifuge Retention Capacity (CRC) of 29.9 g/g, a pH of 5.4, and a Degree of Neutralization (DN) of 57 mole-percent (mol%) was coated at room temperature with a first coating solution (Solution 1) comprising 4 grams of CATIOFAST VHF (a polyvinylamine having a molecular weight of about 200,000 and a solids content of about 22% solids, available from BASF AG, Ludwigshafen, DE) and 2 grams of propylene glycol, followed by a coating with a second coating solution (Solution 2) comprising 0.8 grams of deionized water, 0.8 grams of propylene glycol, 1.4 grams of aluminum sulfate solution (27% solids), and 0.08 grams of ethylene glycol diglycidyl ether (EGDGE), according to the coating procedure described above. The coated base polymer particles were then heated in a BLUE M forced air laboratory oven (available from Thermal Product Solutions, a business having offices located in Montoursville, Pennsylvania, U.S.A.) at 150°C and allowed to cure according to Table 1 below to provide superabsorbent materials of the present invention. The materials were then tested for Centrifuge Retention Capacity (CRC), Absorbency Under Load (AUL) and Gel Bed Permeability (GBP) as described in the test procedures above, the results of which can be seen in Table 1 below.

**Table 1**

| Cure Time (min) | CRC (g/g) | AUL 0.9 psi (6.2 kPa) (g/g) | Free Swell GBP (Darcies) | 0.3 psi (2.1 kPa) GBP (Darcies) |
|---|---|---|---|---|
| 10 | 23.1 | 19.6 | 253 | 21 |
| 20 | 23.6 | 19.5 | 278 | 29 |
| 30 | 23.1 | 19.2 | 274 | 30 |
| 45 | 21.9 | 18.0 | 303 | 43 |
| 60 | 21.6 | 16.5 | 329 | 54 |

### Example 2

In this example, 40 grams of sodium polyacrylate base polymer having a Centrifuge Retention Capacity (CRC) of 23 g/g, a pH of 5.4, and a Degree of Neutralization (DN) of 57 mol% was coated at room temperature with a first coating solution (Solution 1) comprising 4 grams of CATIOFAST VHF (a polyvinylamine having a molecular weight of about 200,000 and a solids content of about 22% solids) and 2 grams of propylene glycol, followed by a coating with a second coating solution (Solution 2) comprising 0.8 grams of deionized water, 0.8 grams of propylene glycol, 1.4 grams of aluminum sulfate solution (27% solids), and 0.08 grams of ethylene glycol diglycidyl ether (EGDGE) according to the coating procedure described above. The coated base polymer particles were then heated in a BLUE M forced air laboratory oven at 150°C and allowed to cure according to Table 2 below to provide superabsorbent materials of the present invention. The materials were then tested for Centrifuge Retention Capacity (CRC), Absorbency Under Load (AUL) and Gel Bed Permeability (GBP) as described in the test procedures above, the results of which can be seen in Table 2 below.

Table 2

| Cure Time (min) | CRC (g/g) | AUL 0.9 psi (6.2 kPa) (g/g) | Free Swell GBP (Darcies) | 0.3 psi (2.1 kPa) GBP (Darcies) |
|---|---|---|---|---|
| 15 | 18.8 | 13.5 | 219 | 21 |
| 30 | 17.0 | 14.8 | 257 | 36 |
| 60 | 13.8 | 11.4 | 229 | 57 |
| 90 | 12.6 | 11.7 | 224 | 69 |

**Comparative Example 1**

In this example, 40 grams of sodium polyacrylate base polymer having a Centrifuge Retention Capacity (CRC) of 30 g/g, a pH of 6.1, and a Degree of Neutralization of 74 mol% was coated at room temperature with a first coating solution (Solution 1) comprising 4 grams of CATIOFAST VHF (a polyvinylamine having a molecular weight of about 200,000 and a solids content of about 22% solids) and 2 grams of propylene glycol, followed by a coating with a second coating solution (Solution 2) comprising 0.8 grams of deionized water, 0.8 grams of propylene glycol, 1.4 grams of aluminum sulfate solution (27% solids), and 0.08 grams of ethylene glycol diglycidyl ether (EGDGE), according to the coating procedure described above. The coated base polymer particles were then heated in a BLUE M forced air laboratory oven at 150°C and allowed to cure according to Table 3 below. The materials were then tested for Centrifuge Retention Capacity (CRC), Absorbency Under Load (AUL) and Gel Bed Permeability (GBP) as described in the test procedures above, the results of which can be seen in Table 3 below.

Table 3

| Cure Time (min) | CRC (g/g) | AUL 0.9 psi (6.2 kPa) (g/g) | Free Swell GBP (Darcies) | 0.3 psi (2.1 kPa) GBP (Darcies) |
|---|---|---|---|---|
| 15 | 23.8 | 18.5 | 205 | 12 |
| 30 | 24.2 | 19.8 | 198 | 14 |
| 60 | 23.9 | 19.4 | 193 | 13 |
| 90 | 24.2 | 18.1 | 228 | 13 |

Examples 1 and 2 and Comparative Example 1, as seen in Tables 1 through 3 show that a polyamine coating on low pH base polymer particles provides an excellent CRC/0.3 psi (2.1 kPa) GBP relationship. In Example 1, a CRC of 23.1 g/g is achieved, with a 0.3 psi (2.1 kPa) GBP of 21 Darcies, which is superior to values expected for a higher pH base polymer (e.g., pH of 6 and DN of 74 mol%) having a polyamine coating, or for a conventional coating on a low pH base polymer. These examples and comparative example also illustrate that the CRC of the polyamine-coated base polymer particles decreases only slightly, while the 0.3 psi (2.1 kPa) GBP increases dramatically at longer curing times. An excellent CRC/GBP relationship is obtained, as demonstrated by the fact that fluid absorption is retained while permeability is substantially increased. In comparison, Comparative Example 1 resulted in a poor permeability (i.e., a low 0.3 psi (2.1 kPa) GBP). As illustrated below, the base polymer of Example 3 (pH of 5.7, DN of 60%) exhibits a similar improved performance with greater insensitivity to curing time and temperature.

**Comparative Example 2**

In this example, 100 weight parts of polyacrylic acid base polymer having a Centrifuge Retention Capacity (CRC) of 33 g/g, a pH of 5.7, and a Degree of Neutralization (DN) of 60 mol% were coated at room temperature with 7.5 weight parts of a coating solution comprising 27% by weight water, 27% by weight propylene glycol, and 46% by weight aluminum sulfate solution (27% $Al_2(SO_4)_3$) according to the coating procedure described above. Ethylene glycol diglycidyl ether (EGDGE) surface crosslinking agent was also added to the coating solution in various amounts according to Table 4 below. The coated base polymer particles then were placed in a BLUE M forced air laboratory oven at 130°C and cured for 1 hour. The materials were then tested for Centrifuge Retention Capacity (CRC) and Gel Bed Permeability (GBP) as described in the test procedures above, the results of which can be seen in Table 4 below.

**Table 4**

| EGDGE (ppm) | CRC (g/g) | 0.3GBP (Darcies) |
|---|---|---|
| 0 | 33 | 0 |
| 200 | 27.6 | 1.3 |
| 400 | 27.5 | 1.7 |
| 600 | 26.5 | 3.1 |
| 600 | 25.8 | 3.4 |
| 1200 | 24.6 | 8 |
| 1800 | 23.7 | 8.4 |
| 2400 | 24.2 | 10.8 |

## Example 3

In this example, 100 weight parts of the base polymer used in Comparative Example 2 were coated at room temperature with 7.5 weight parts of a solution comprising 61% LUPAMIN 9095 (a polyvinylamine of molecular weight 200,000, available from BASF AG, Ludwigshafen, DE), 19.5% water and 19.5% propylene glycol according to the coating procedure described above. The treated base polymer particles were then further coated with 7.5 weight parts of a solution containing 1.36% EGDGE, 27.44% water, 27.4% propylene glycol and 43.8% aluminum sulfate solution (27% $Al_2(SO_4)_3$) according to the coating procedure described above. The resulting coated base polymer particles were then placed in a BLUE M forced air laboratory oven at 110°C and were allowed to cure for 70 minutes to provide superabsorbent materials of the present invention. These superabsorbent materials were then tested for Centrifuge Retention Capacity (CRC) and Gel Bed Permeability (GBP) as described in the test procedures above, and resulted in a CRC of 25.5 g/g and a 0.3 GBP of 15.0 Darcies.

It can be seen that the permeability at similar capacities is much lower for the particles of Comparative Example 2 than for the particles of Example 3. Even at much lower capacities the comparative particles have an inferior permeability compared to the polyamine-coated superabsorbent materials of the present invention.

## Comparative Example 3

In this example, 100 weight parts of polyacrylic acid base polymer particles having a Centrifuge Retention Capacity (CRC) of 33 g/g, a pH of 6.0, and a Degree of Neutralization (DN) of 74 mol% were coated at room temperature with 7.5 weight parts of a solution comprising 27% water, 27% propylene glycol and 46% aluminum sulfate solution (27% $Al_2(SO_4)_3$) according to the coating procedure described above. Ethylene glycol diglycidyl ether (EGDGE) surface crosslinking agent was also added to the coating solution in various amounts as seen in Table 5 below. The resulting coated base polymer particles were then placed in a BLUE M forced air laboratory oven at 130°C and were allowed to cure for 1 hour. The materials were then tested for Centrifuge Retention Capacity (CRC) and Gel Bed Permeability (GBP) as described in the test procedures above, the results of which can be seen in Table 5 below.

**Table 5**

| EGDGE (ppm) | CRC (g/g) | 0.3GBP (Darcies) |
|---|---|---|
| 0 | 33 | 0 |
| 375 | 29.65 | 2.33 |
| 750 | 28.24 | 3.72 |
| 1125 | 27.85 | 5.23 |
| 1500 | 27.03 | 8.82 |

## Example 4

In this example, 100 weight parts of polyacrylic acid base polymer particles having a Centrifuge Retention Capacity (CRC) of 33 g/g, a pH of about 5.8, and a Degree of Neutralization (DN) of 67 mol% were coated at room

temperature with 7.5 weight parts of a solution comprising 61 % LUPAMIN 9095, 19.5% water, and 19.5% propylene glycol according to the coating procedure described above. The treated base polymer particles then were further coated with 7.5 parts of a solution containing 1.36% EGDGE, 27.44% water, 27.4% propylene glycol, and 43.8% aluminum sulfate solution (27% $Al_2(SO_4)_3$) according to the coating procedure described above. The resulting coated base polymer particles were then place in a BLUE M forced air laboratory oven at 110°C and were allowed to cure for 70 minutes to provide superabsorbent materials (SAM) of the present invention. The resulting superabsorbent materials were tested for Centrifuge Retention Capacity (CRC) and Gel Bed Permeability (GBP) as described in the test procedures above, and resulted in a CRC of 26.2 g/g and a 0.3 GBP of 6.9 Darcies. Additional samples were also made wherein the amounts of LUPAMIN 9095 and EGDGE were varied, the results of which can be seen in Table 6 below.

**Table 6**

| % LUPAMIN 9095 (based on solids) | % Aluminum Sulfate (based on SAM) | EGDGE (ppm SAM) | CRC (g/g) | 0.3 GBP (Darcies) |
|---|---|---|---|---|
| 1 | 0.84 | 1000 | 26.2 | 6.9 |
| 1 | 0.84 | 2000 | 24.5 | 12.3 |
| 1 | 0.85 | 3000 | 23.4 | 13.7 |
| 1.5 | 0.84 | 1000 | 26.2 | 8 |
| 1.5 | 0.84 | 2000 | 23.6 | 19.2 |

It can be seen that higher amounts of EGDGE resulted in superabsorbent materials having increased Gel Bed Permeabilities.

**Example 5**

In this example, 50 grams of sodium polyacrylate base polymer having a Centrifuge Retention Capacity (CRC) of 35.3 g/g and a Degree of Neutralization (DN) of 60 mol% were coated at room temperature with 3.75 grams of a first coating solution (Solution 1) comprising 2.38 grams of LUPAMIN 9095, 0.73 grams of propylene glycol and 0.73 grams of deionized water according to the coating procedure described above. This first coating was immediately followed by coating with a second solution (Solution 2) comprising 0.73 grams of water, 0.73 grams of propylene glycol, 0.05 grams of ethylene glycol diglycidyl ether, and a quantity of 27% aluminum sulfate solution (see Table 6 below) according to the coating procedure described above. The coated base polymer particles were then placed in a BLUE M forced air laboratory oven at 150°C and were allowed to cure for 1 hour to provide superabsorbent materials of the present invention. The materials were then tested for Centrifuge Retention Capacity (CRC) and Gel Bed Permeability (GBP) as described in the test procedures above, the results of which can be seen in Table 7 below.

**Table 7**

| Aluminum Sulfate (% Based on SAM) | CRC (g/g) | 0.3 GBP (Darcies) |
|---|---|---|
| 1.00 | 26.5 | 12.9 |
| 0.75 | 26.3 | 12.2 |
| 0.50 | 27.1 | 6.7 |
| 0.25 | 27.7 | 4.5 |
| 0.00 | 28.8 | 4.8 |

It can be seen that higher amounts of Aluminum Sulfate resulted in improved Gel Bed Permeabilities, while substantially maintaining Centrifuge Retention Capacity.

**Example 6 - Substantially Uniform Absorbent Core Structure**

In this example, absorbent core structures were formed comprising a substantially uniform distribution of superabsorbent materials and fluff fibers. Superabsorbent materials of the present invention were prepared as follows:

**SAM-1**

The superabsorbent material (SAM-1) was prepared by placing 100 parts of poly(sodium acrylate) base polymer having a Centrifuge Retention Capacity of 34 g/g and a Degree of Neutralization of 60 mol% into a Lödige M5 mixer (available from Gebruder Lödige Maschinenbau GmbH, a business having offices located in Paderborn, Germany) running at 300 rpm at room temperature. The mixer was turned on and 7.5 parts of a first coating solution (Solution 1) comprising 26.7% by weight water, 26.7% by weight propylene glycol, 45.8% by weight alum solution (27% aluminum sulfate) and 0.8% by weight EGDGE was injected into the mixer using a syringe and was allowed to coat the particles. The mixer was turned off and the coated base polymer particles were cured in a BLUE M forced air laboratory oven at 130°C for 60 minutes. The coated particles were then placed back into the mixer, and the mixer was turned on. Then 8 parts of a second coating solution (Solution 2) comprising 60.8% by weight LUPAMIN 9095 (a polyvinylamine of molecular weight 200,000, available from BASF AG, Ludwigshafen, DE), 38.8% by weight propylene glycol, and 0.4% by weight EGDGE was injected into the mixer using a syringe and was also allowed to coat the particles. The mixer was turned off, and the coated base polymer particles were then cured in a BLUE M forced air laboratory oven at 70°C for 60 minutes to provide superabsorbent materials of the present invention. The superabsorbent materials exhibited a Centrifuge Retention Capacity (CRC) of 24.1 g/g and a 0.3 (2.1 kPa) psi Gel Bed Permeability (GBP) of 0.9 Darcies, according to the test procedures described above.

**Substantially Uniform Absorbent Core**

Handsheets were prepared using standard airforming handsheet equipment to yield a 25.4 by 43.2cm (10 inch by 17 inch) composite handsheet. A total of 40.31 grams of each superabsorbent material described above and 17.27 grams of NB480 fluff fiber were used to create each sample with a target basis weight of 500 grams per square meter (GSM). Forming tissue with a basis weight of 16.6 gsm (White Wrap Sheet, available from Cellu Tissue Holdings, Inc., a business having offices located in East Hartford, Connecticut, U.S.A.) was used for the top and bottom of the samples. A sheet of the forming tissue was placed on the bottom of the former. The superabsorbent material and the NB480 were each divided into equal portions (6 portions of fluff and 5 portions of superabsorbent materials). Each fluff portion was then introduced into the top of the former, followed immediately with a superabsorbent portion, allowing the vacuum to disperse the fluff and superabsorbent materials into the former chamber and onto the forming tissue. This alternating process was continued until the last portion of fluff was consumed, forming a substantially uniform distribution of fluff and superabsorbent materials. A comparative example was also made by substituting HYSORB 8800 AD (a conventional superabsorbent material (i.e., non-polyamine coated material) available from BASF) for the superabsorbent material of the present invention. Another sheet of forming tissue was then placed on top of the sample, and the sample was placed into a CARVER PRESS model #4531 (available from Carver, Inc., a business having offices located in Wabash, Indiana, U.S.A.) and densified to approximately 0.35 g/cc. Samples were then cut from the composite handsheet in appropriate sizes for testing, as set forth in the test procedures described above. The samples were then tested for Saturated Capacity (SAT CAP) and Intake Rate as described in the test procedures above, the results of which can be seen in Table 8 below.

**Table 8**

| SAP | 0.5psi (3.4 kPa) Saturated Capacity (g/g) | $2^{nd}$ Insult Intake Rate (ml/sec) | $3^{rd}$ Insult Intake Rate (ml/sec) |
|---|---|---|---|
| Hysorb 8800AD (Comparative Example) | 24.2 | 1.07 | 0.77 |
| SAM-1 | 22.6 | 1.35 | 1.09 |

**Example 7 - Layered Absorbent Core Structure**

In this example, absorbent core structures were formed, each having a layer comprising substantially superabsorbent materials sandwiched between two layers comprising substantially fluff fibers. Superabsorbent materials of the present invention were prepared as described in Example 6 above. Layered absorbent core samples were then prepared using standard airforming handsheet equipment to yield a 25.4 by 43.2 cm (10 inch by 17 inch) composite handsheet. A total of 40.31 grams of each superabsorbent materials of the present invention and 17.27 grams of NB480 fluff fiber were used to create each sample, with a target basis weight of 500 grams per square meter (GSM). Forming tissue with a basis weight of 16.6 gsm (White Wrap Sheet, available from Cellu Tissue Holdings, Inc.) was used for the top and bottom of the samples. The fiber and superabsorbent materials were divided into two equal parts, approximately

8.64 grams and 20.16 grams each, respectively. A sheet of forming tissue was placed on the bottom of the former. One part of the fiber was introduced to the top of the former allowing the vacuum to disperse it into the former chamber and onto the forming tissue. After all of the fiber had been deposited, one part of the superabsorbent materials was introduced gradually to achieve uniform distribution. Then the second part of the superabsorbent materials was added gradually directly following the first part. Once all of the superabsorbent materials were deposited, the second part of the fiber was introduced, creating the layered composite. A comparative example was also made by substituting HYSORB 8800 AD (a conventional superabsorbent material (i.e., non-polyamine coated material) available from BASF) for the superabsorbent material of the present invention. Another sheet of forming tissue was then placed on top of the sample, and the sample was placed into a CARVER PRESS model #4531 and densified to approximately 0.35 g/cc. Samples were then cut from the composite handsheets in appropriate sizes for testing as set forth in the test procedures described above. The samples were then tested for Saturated Capacity (SAT CAP) and Intake Rate as described in the test procedures above, the results of which can be seen in Table 9 below.

**Table 9**

| SAP | 0.5psi (3.4 kPa) Saturated Capacity (g/g) | 2$^{nd}$ Insult Intake Rate (ml/sec) | 3$^{rd}$ Insult Intake Rate (ml/sec) |
|---|---|---|---|
| Hysorb 8800AD (Comparative Example) | 23.6 | 0.80 | 0.52 |
| SAM-1 | 23.3 | 2.14 | 1.86 |

From Tables 8 and 9, it can be seen that absorbent structures comprising the coated superabsorbent material of the present invention (i.e., SAM-1) generally exhibit an improved 2$^{nd}$ and 3$^{rd}$ Insult Intake Rate at a similar Saturated Capacity when compared to conventional superabsorbent materials. Additionally, when the superabsorbent materials of the present invention were incorporated into the layered absorbent core structure, the 2$^{nd}$ and 3$^{rd}$ Insult Intake Rates improved (i.e., increased) when compared to the homogeneously mixed absorbent core structure. However, when the conventional superabsorbent material was incorporated into the layered absorbent core structure, the 2$^{nd}$ and 3$^{rd}$ Insult Intake Rates decreased. Thus, an additional improvement may be gained by incorporating the superabsorbent materials of the present invention into a layered absorbent core structure.

It will be appreciated that details of the foregoing examples, given for purposes of illustration, are not to be construed as limiting the scope of this invention. Although only a few exemplary embodiments of this invention have been described in detail above, those skilled in the art will readily appreciate that many modifications are possible in the examples without materially departing from the novel teachings and advantages of this invention. For example, features described in relation to one example may be incorporated into any other example of the invention.

Accordingly, all such modifications are intended to be included within the scope of this invention, which is defined in the following claims and all equivalents thereto. Further, it is recognized that many embodiments may be conceived that do not achieve all of the advantages of some embodiments, particularly of the preferred embodiments, yet the absence of a particular advantage shall not be construed to necessarily mean that such an embodiment is outside the scope of the present invention. As various changes could be made in the above constructions without departing from the scope of the invention, it is intended that all matter contained in the above description shall be interpreted as illustrative and not in a limiting sense.

## Claims

**1.** An absorbent article comprising:

a topsheet;
a backsheet; and
an absorbent core disposed between said topsheet and said backsheet; wherein
said absorbent core comprises a superabsorbent material that includes a base polymer; wherein
said base polymer has a pH less than six and a surface coating comprising a polyamine.

**2.** The absorbent article of claim 1 wherein said surface coating further comprises an inorganic salt having a polyvalent metal cation.

**3.** The absorbent article of claim 2 wherein said inorganic salt has a water solubility of at least 0.1 grams per 100 grams

of water at 25°C.

4. The absorbent article of claim 2 or claim 3 wherein said polyvalent metal cation has a valence in the range of +2 to +4.

5. The absorbent article of any of claims 2 to 4 wherein said polyvalent metal cation is selected from $Mg^{2+}$, $Ca^{2+}$, $Al^{3+}$, $Sc^{3+}$, $Ti^{4+}$, $Mn^{2+}$, $Fe^{2+}$, $Fe^{3+}$, $Co^{2+}$, $Ni^{2+}$, $Cu^{+/2+}$, $Zn^{2+}$, $Y^{3+}$, $Zr^{4+}$, $La^{3+}$, $Ce^{4+}$, $Hf^{4+}$, $Au^{3+}$, and mixtures thereof.

6. The absorbent article of any of claims 1 to 5 wherein said superabsorbent material is surface crosslinked.

7. The absorbent article of any of claims 1 to 6 wherein said base polymer has a pH between 4 and 6.

8. The absorbent article of claim 7 wherein said base polymer has a pH between 5 and 6.

9. The absorbent article of any of claims 1 to 8 wherein said base polymer comprises a plurality of pendant neutralized and unneutralized carboxylic acid groups.

10. The absorbent article of any of claims 1 to 9 wherein said base polymer is selected from acrylic acid, methacrylic acid, and combinations thereof.

11. The absorbent article of any of claims 1 to 10 wherein said polyamine is present on surfaces of said base polymer in an amount of about 0.1% to about 2% by weight of said base polymer.

12. The absorbent article of any of claims 1 to 11 wherein said polyamine comprises at least one selected from primary amino groups, secondary amino groups, tertiary amino groups, and quaternary ammonium groups.

13. The absorbent article of any of claims 1 to 12 wherein said polyamine has a weight average molecular weight of about 5,000 to about 1,000,000.

14. The absorbent article of any of claims 1 to 13 wherein said polyamine is selected from polyvinylamine, polyethyleneimine, polyallylamine, polyalkyleneamine, polyazetidine, polyvinylguanidine, poly(DADMAC), cationic polyacrylamide, polyamine functionalized polyacrylate, and combinations thereof.

15. The absorbent article of any of claims 1 to 13 wherein said polyamine is a copolymer of polyvinylamine, polyethyleneimine, polyallylamine, polyalkyleneamine, polyazetidine, polyvinylguanidine, poly(DADMAC), cationic polyacrylamide, or polyamine functionalized polyacrylate, and combinations thereof.

16. The absorbent article of any of claims 1 to 15 wherein said base polymer comprises a polyacrylic acid.

17. The absorbent article of any of claims 1 to 16 wherein said base polymer has an internal crosslinking density between about 0.01% and about 7%.

18. The absorbent article of any of claims 1 to 17 wherein said base polymer is surface crosslinked.

19. The absorbent article of any of claims 1 to 18 wherein at least one of said topsheet, backsheet, and absorbent core is stretchable.

20. The absorbent article of any of claims 1 to 19 wherein said absorbent core comprises layers.

21. The absorbent article of claim 20 wherein at least one of said layers comprises substantially said superabsorbent material and at least one of said layers comprises substantially fluff.

22. The absorbent article of claim 20 or claim 21 wherein said absorbent core exhibits a greater $2^{nd}$ and $3^{rd}$ Insult Intake Rate when compared to a homogenous absorbent core.

23. The absorbent article of any of claims 1 to 22 wherein said article is selected from the group consisting of personal care absorbent articles, health/medical absorbent articles, and household/industrial absorbent articles.

24. The absorbent article of any of claims 1 to 23 wherein said absorbent core comprises at least about 10% by weight

of said superabsorbent materials.

**25.** The absorbent article of any of claims 1 to 23 wherein said absorbent core comprises at least about 30% by weight of said superabsorbent materials.

**26.** The absorbent article of any of claims 1 to 23 wherein said absorbent core comprises at least about 60% by weight of said superabsorbent materials.

**27.** The absorbent article of any of claims 1 to 23 wherein said absorbent core comprises between about 10% and about 80% by weight of said superabsorbent materials.

**28.** The absorbent article of any of claims 1 to 27 wherein said absorbent core further comprises fluff.

**29.** The absorbent article of any of claims 1 to 28 wherein said absorbent core further comprises a surfactant.

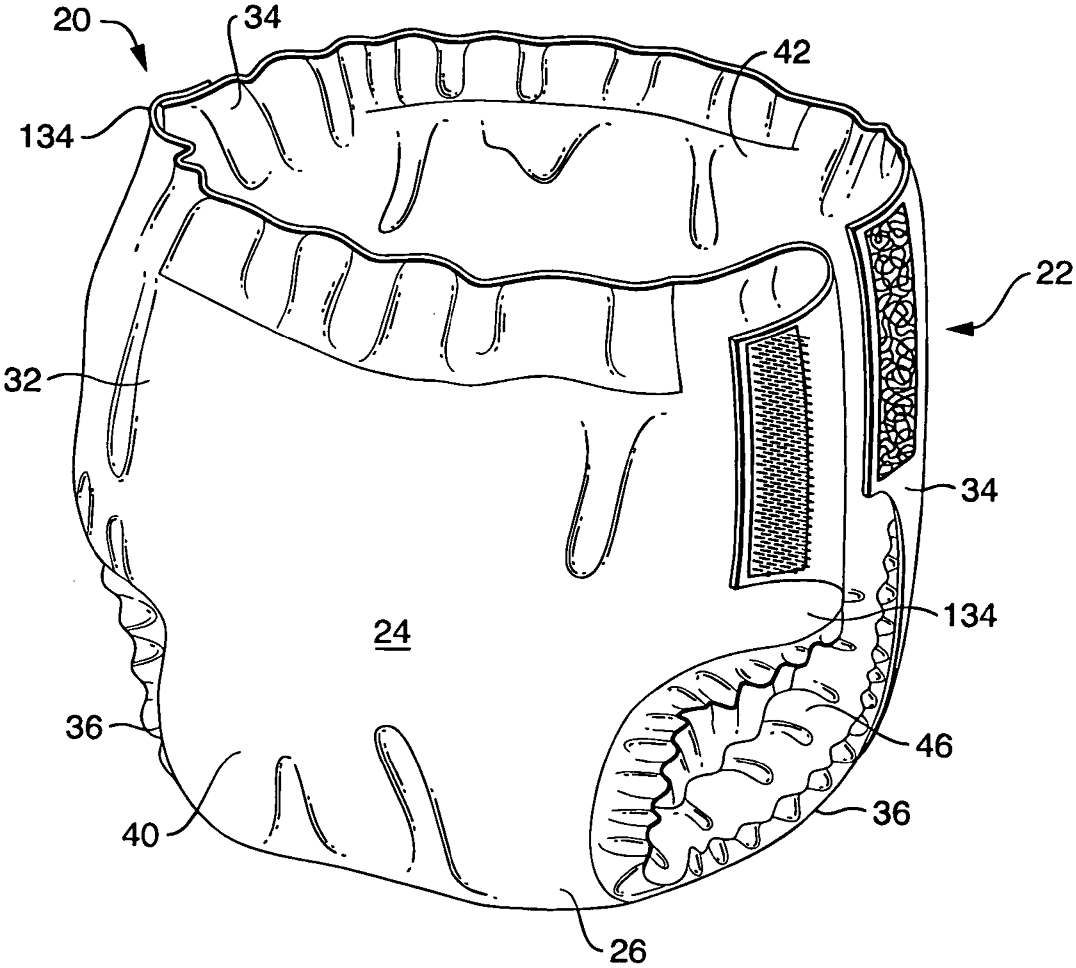


FIG. 1

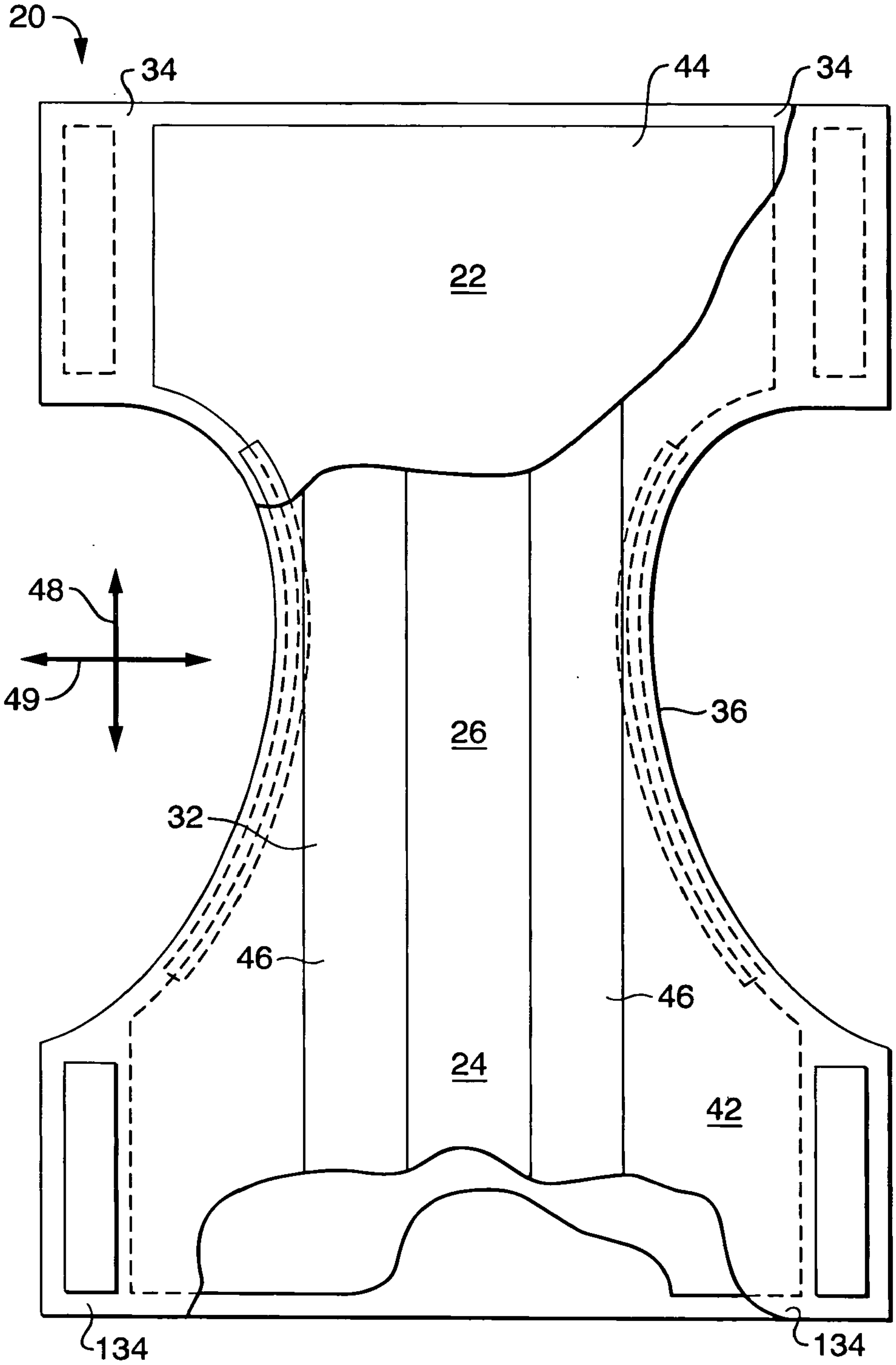


FIG. 2

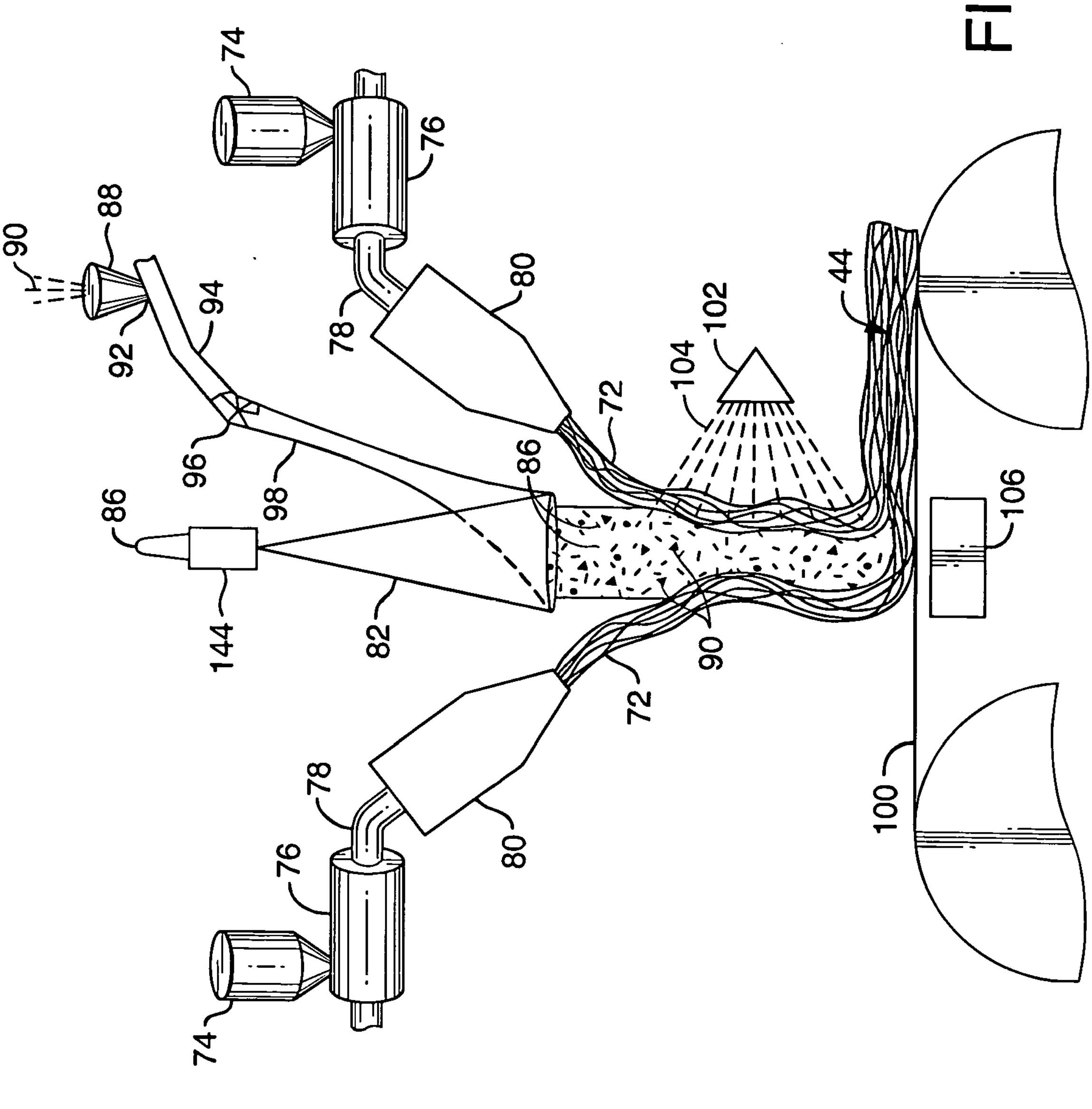
74
76
90
88
78
80
94
92
102
44
104
72
96
86
98
86
144
82
90
106
72
78
100
80
76
74

FIG. 3

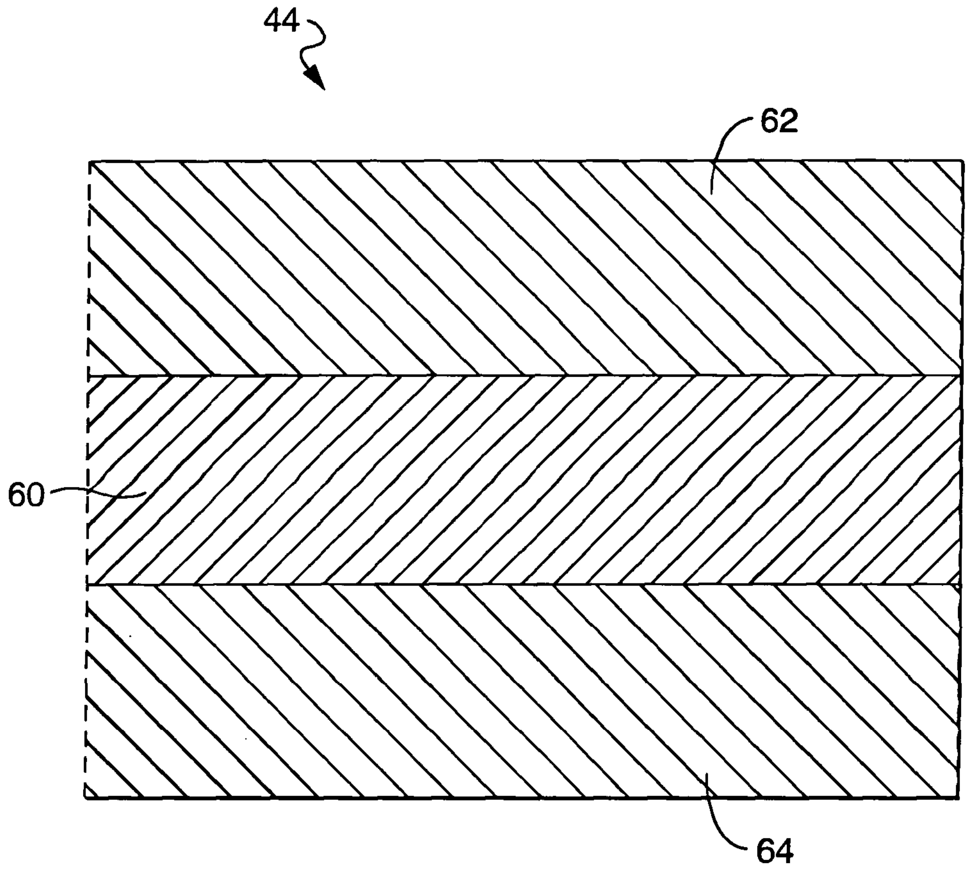


FIG. 4

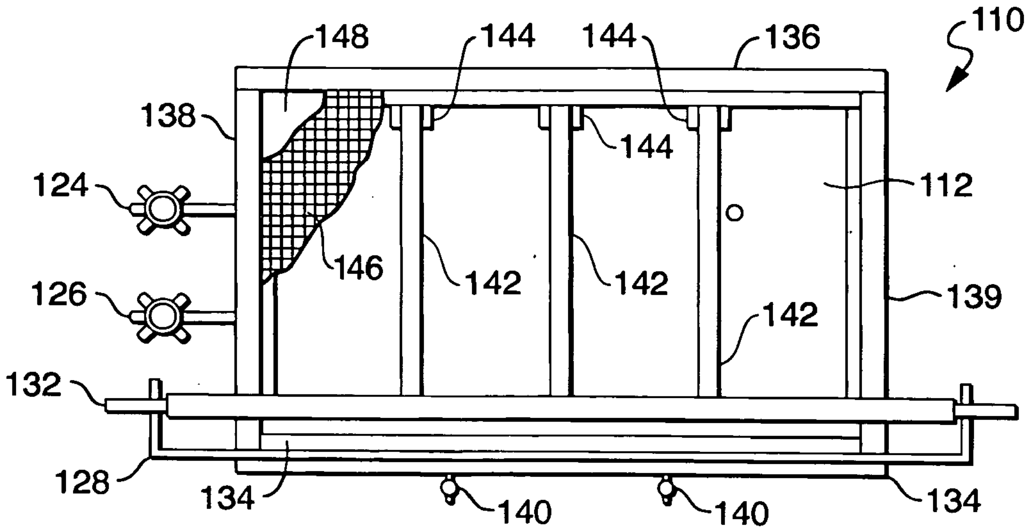
110
148
144
144
136
138
124
126
112
146
142
142
142
139
132
128
134
140
140
134

FIG. 5

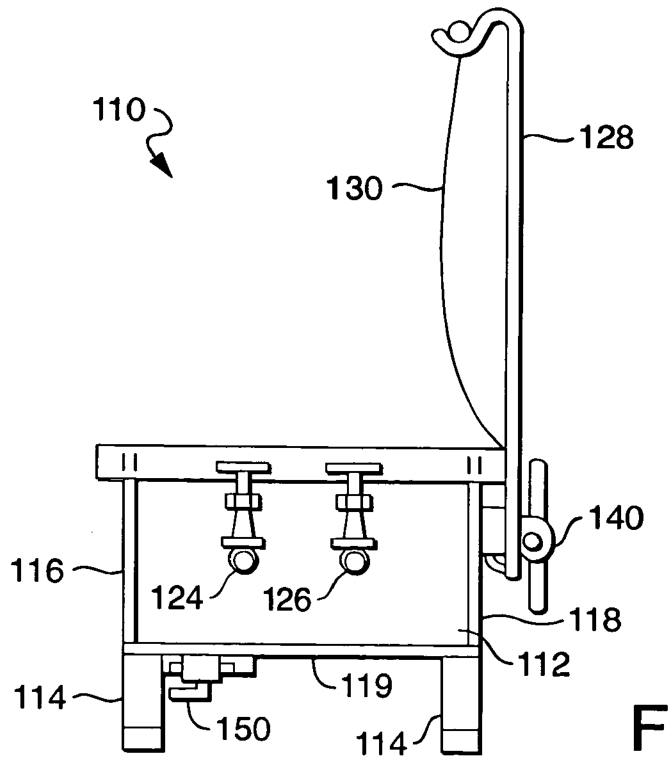
110
128
130
140
116
124
126
118
112
119
114
150
114

FIG. 6

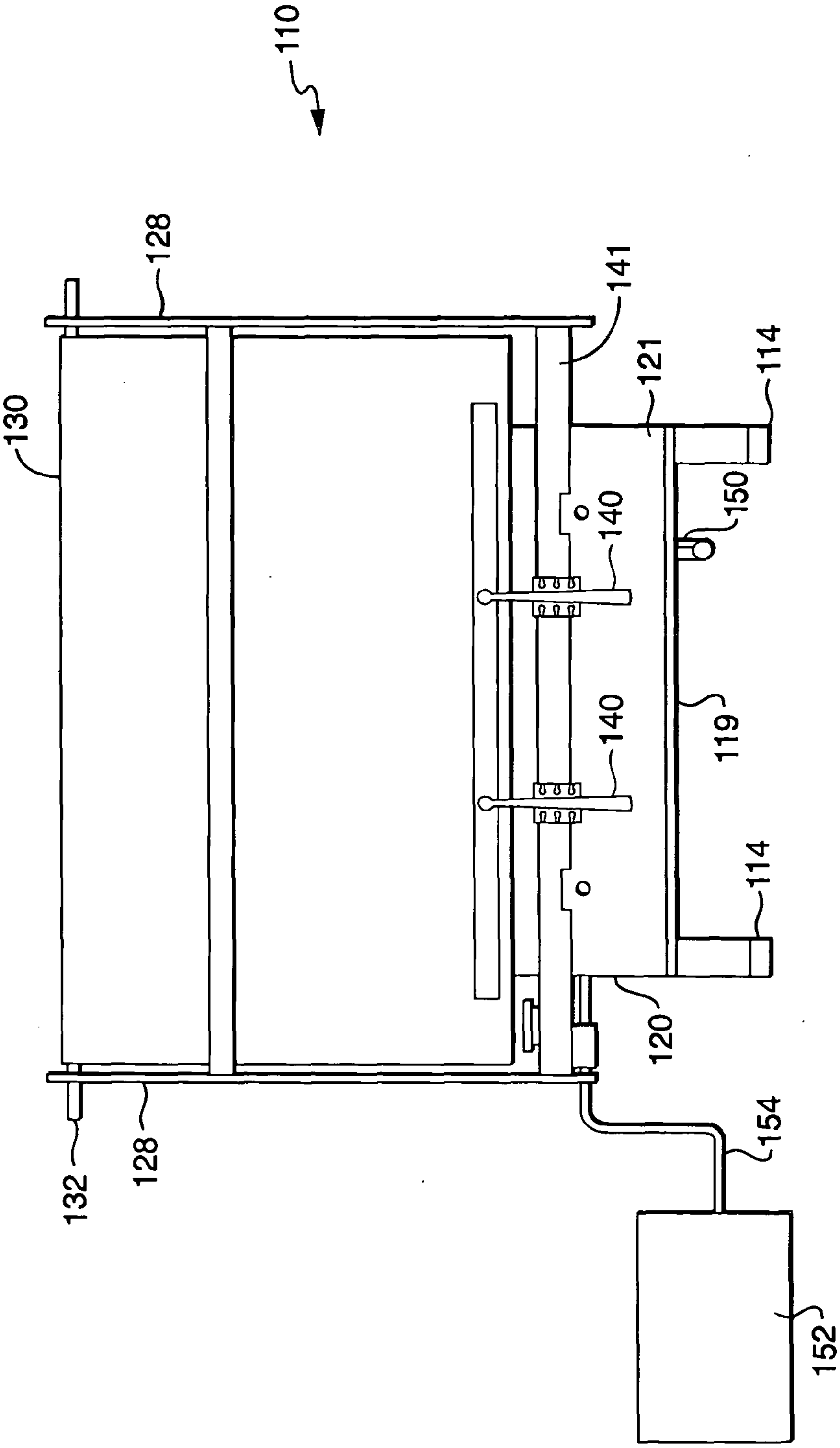


FIG. 7

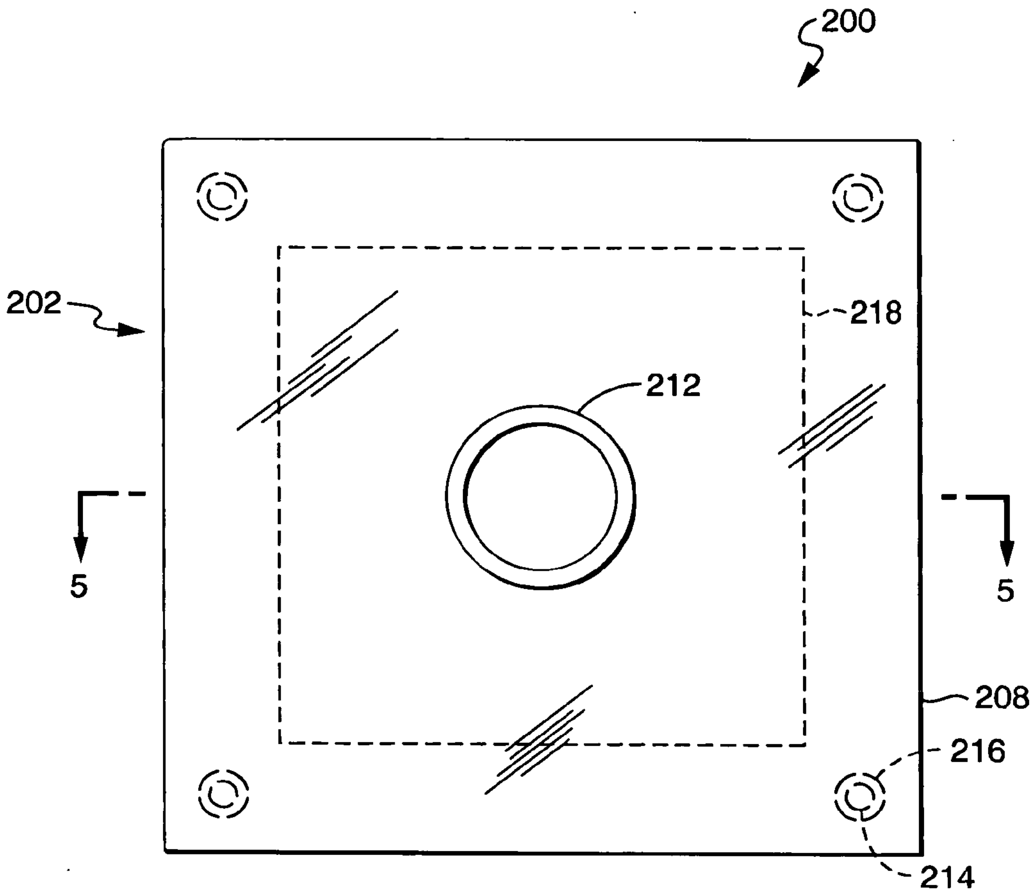
200
202
218
212
5
5
208
216
214

FIG. 8

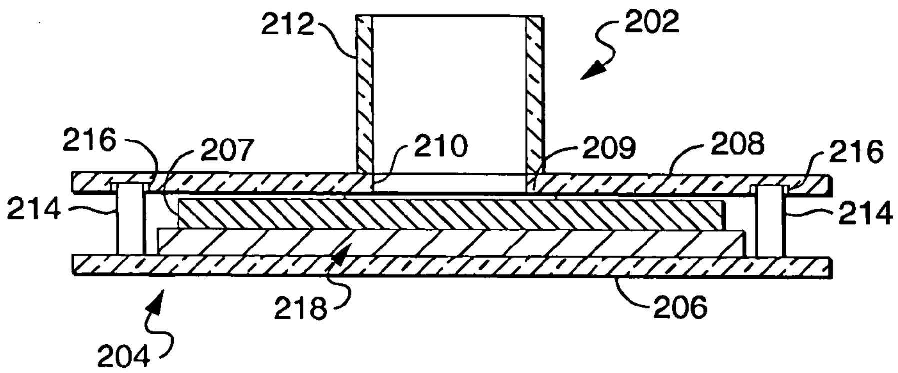
212
202
216
207
210
209
208
216
214
214
218
206
204

FIG. 9